(19) Europäisches Patentamt / European Patent Office / Office européen des brevets

(11) **EP 3 984 527 A1**

(12) **EUROPEAN PATENT APPLICATION**

(43) Date of publication:
**20.04.2022  Bulletin 2022/16**

(21) Application number: **20201629.1**

(22) Date of filing: **13.10.2020**

(51) International Patent Classification (IPC):
**A61K 9/16** (2006.01)     **A61K 47/69** (2017.01)

(52) Cooperative Patent Classification (CPC):
**A61K 9/1623; A61K 9/1694; A61K 9/5123;
A61K 47/6929; A61K 47/6935**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Ludwig-Maximilians-Universität
München
80539 München (DE)**

(72) Inventors:
• **MERKEL, Olivia
  82178 Puchheim (DE)**
• **KEIL, Tobias
  81475 München (DE)**
• **ZIMMERMANN, Christoph
  80799 München (DE)**

(74) Representative: **Patent- und Rechtsanwälte
Behrmann Wagner
PartG mbB
Hegau-Tower
Maggistraße 5 (11. OG)
78224 Singen (DE)**

(54) **NANO-IN-MICRO ENCAPSULATED SIRNA DRY POWDER, METHOD FOR PRODUCING THE SAME AND USE OF A POWDER FORMULATION AS A PHARMACEUTICAL DOSAGE FORM, IN PARTICULAR FOR PULMONARY DELIVERY**

(57)     Method for producing high yield nano-in-micro (NIM) encapsulated bioactive siRNA dry powder comprising the steps of:
- providing an aqueous suspension comprising polyplexes, in particular polyelectrolyte complexes, formed from at least a polyamine, and/or polyamide and/or polyester and siRNA, wherein the polyplexes are provided with and/or encapsulated into water soluble excipients, in particular highly purified water and/or sugar alcohol and/or sugar;
- spray drying the aqueous suspension using a spray drying apparatus, preferably a Büchi B-290, by feeding the aqueous suspension to a spray drying atomizing nozzle and subjecting the atomized droplets to a heated gas stream of a carrier gas, preferably dried and cleaned air, in particular through a multicomponent atomizing nozzle for the suspension and an atomizing gas;
- collecting a spray dried powder in an accumulation means of the spray drying apparatus
characterized in that
the temperature in the vicinity of an outlet opening of the spray drying apparatus, in particular an outlet opening connecting a spray drying chamber with the accumulation means, during feeding of the aqueous suspension to the nozzle is controlled by temperature controlling means to be limited to an upper threshold temperature which is equal to or below a melting temperature of the respective naked siRNA.

Fig. 11

EP 3 984 527 A1

**Description**

The current invention relates to a method for producing a Nano-in-Micro (NIM) encapsulated siRNA dry powder according to claim 1. The invention further relates to a powder comprising Nano-in-Micro encapsulated siRNA according to claim 8. The invention further relates to a use of a powder composition or blend as a pharmaceutical composition, in particular for pulmonary delivery.

Application of drugs directly to their site of action is the optimal way to reduce doses and side effects. For lung diseases such as asthma, pulmonary delivery is therefore favored. [1] With a relatively low enzyme activity and a slow surface clearance [2], enzymatically prone substances are perfect candidates for this administration route. In addition, dry powder inhalers enable the delivery of drugs with a high shelf life and also provide an easy to use tool for patients along with high compliance. [3] Despite several available treatments for lung diseases, gene therapy is a promising new tool to address uncontrollable disease variants such as severe, uncontrolled asthma, but also particularly viruses for which no antiviral compounds are available. Knowing the genome of the virus is sufficient to develop nucleic acid based therapeutics that can inhibit viral replication. [4] Small interfering RNA (siRNA) can silence the translation of messenger RNA into pathologically upregulated proteins and diminish disease symptoms. [5] However, siRNA therapeutics face several challenges associated with the delivery into cells and enzymatic stability. To address these issues, nanoparticles are preferred to protect and encapsulate siRNA.

Numerous vehicles are reported in literature to achieve uptake and transfection of cells with siRNA. [6] However, the only clinically approved siRNA drugs target the liver, and pulmonary delivery is still in the future. [7, 8]

Cationic polymers are one class of nucleic acid nanocarriers amongst which polyethylenimine (PEI) is the most studied one. However, the use of PEI is limited due to its cytotoxicity profile. To overcome toxic characteristics of PEI the applicant has developed copolymers of PEI with better safety profiles. These copolymers combine the nucleic acid condensation and protection efficiency of PEI but also the transfection and stability effects of polycaprolactone (PCL) and polyethyleneglycol (PEG), respectively. It was shown that these copolymers of PEG-PCL-PEI (PPP) or Transferrin conjugated polyethylenimine (Tf-PEI) can form so called polyplexes with nucleic acids in the nanoscale by electrostatic interaction and successfully transfect cells *in vitro* and *in vivo* [9-11]. In order to deliver these nanoparticles to the lung, incorporation into microparticles with aerodynamic diameters between 1 and 5 μm is required. The matrices of these microparticles need to consist of excipients which readily dissolve upon impact on lung lining fluid and release their nano cargo. [12] The use of water soluble substances is hence required.

A known technique to produce such nano-in-microparticles (NIM) is spray drying. It is a widely applied method in food, cosmetic, chemical and pharmaceutical industry and allows gentle drying of small droplets. [13] In addition, it is much faster and less time and energy consumptive than spray freeze drying which is also used for the production of inhalable dry powder formulations of siRNA therapeutics. [14] However, as spray drying applies heat to samples, the process might degrade or inactivate siRNA and/or the nucleic acid nanocarrier, such as PPP or Tf-PEI. [12] As the absolute and relative amount of bioactive siRNA in a dry powder formulation and its reliable reproduction, in particular in large scale applications is a critical parameter not only with respect to the pharmaceutical approval process but also with respect to the economic production of such dry powders and pharmaceutical compositions, it is important to establish a spray drying process and a resulting dry powder, that exhibits the desired geometric properties of particle size and distribution with a reliably high percentage of successfully encapsulated and bioactive siRNA.

Therefore, the aim of the invention is to suggest a method for producing a dry powder comprising bioactive nano-in-micro encapsulated siRNA with high quantity and integrity, leading to a high degree of redispersability of the siRNA nanoparticles.

This problem is solved by a method according to independent claim 1 for producing high yield nano-in-micro (NIM) encapsulated bioactive siRNA dry powder comprising the steps of:

- Providing an aqueous suspension comprising polyplexes, in particular polyelectrolyte complexes, formed from at least a polyamine, polyamide and/or polyester and siRNA, wherein the polyplexes are encapsulated into water soluble excipients, in particular highly purified water and/or sugar alcohol and/or sugar;
  spray drying the aqueous suspension using a spray drying apparatus, preferably a Büchi B-290, by feeding the aqueous suspension to a spray drying atomizing nozzle and subjecting the atomized droplets to a heated gas stream of a carrier gas, preferably dried and cleaned air, in particular through a multicomponent atomizing nozzle for both the suspension and gas and atomizing gas;
- collecting a spray dried powder in an accumulation means of the spray drying apparatus
  characterized in that,
  the temperature in the vicinity of an outlet opening of the spray drying apparatus, in particular an outlet opening connecting a spray dry chamber with the accumulation means, during feeding of the aqueous suspension to the nozzle is controlled by temperature controlling means to be limited to an upper threshold temperature which is equal to or below a melting temperature of naked siRNA.

The basic idea of the invention relies on quantification of siRNA upon polyplex redispersion after spray drying depending on spray drying parameters, especially controlling the outlet temperature, and on preserving nanoparticle characteristics of spray dried materials. It was found that the outlet temperature plays an important role for reducing the loss of bioactive siRNA even though the temperature of the inlet, which may also be monitored and which is normally the temperature that is limited to thereby control the outlet temperature is significantly higher.

Surprisingly, outlet temperatures that were above the temperatures for which the respective naked siRNA was known to melt, led to significantly increased loss of bioactive or functional siRNA after spray drying and possible redispersion compared to results obtained by the use of lower outlet temperatures. The outlet temperature is, as a rule of thumb, similar or slightly above the temperature that a powder or dried solution/suspension is heated to during known spray drying processes. However, it is surprising that even when combined with a polyamide and/or polyamine and/or polyester and encapsulated into a matrix of water soluble excipients, the siRNA is neither thermally shielded nor chemically stabilized during the spray drying to a significant or measurable degree, being reflected in an outlet temperature above the melting temperature of the naked siRNA that would not lead to significantly increased losses of bioactive siRNA after the spray drying. It is especially advantageous that by the inventive method dry powder compositions were generated that exhibited the particle size distributions which are necessary for pharmaceutical use, especially pulmonary delivery.

Mannitol can be used as a sugar alcohol. Bur other sugar alcohols are equally suitable candidates. Trehalose can be used as a sugar. Again other sugars can be used as well.

Thus, it is an advantageous embodiment of the invention to control temperature in the vicinity of an outlet opening of the spray drying apparatus or the outlet temperature or upper threshold temperature to a value that is within a range of 5°C, in particular in the range of 3°C, under/below the melting temperature of the respective naked siRNA.

According to a further advantageous embodiment of the invention the upper threshold temperature or outlet temperature is set to 90°C, in particular 80°C. For different siRNA sequences this is a temperature that allows high yield of bioactive siRNA while reaching the low residual moisture and a desired particle size and size distribution of the dry powder.

In a further useful embodiment of the invention, the aqueous suspension is fed to the nozzle through a low abrasive tubing material, in particular a low abrasive silicon based tubing material, especially Pumpsil ®. It was surprisingly found that the tubing material has a significant effect on the amount or percentage of bioactive siRNA in the spray dried powder. In particular, if the temperature is controlled as given above and the aqueous suspension is fed to the nozzle through a low abrasive tubing material, the losses of siRNA can be reduced to less than 10% of the siRNA content, i.e. less than 10% of the mass of the original siRNA used, especially less than 5% of the siRNA content or mass. It is especially advantageous, that the low abrasive tubing material has no negative effect on the spray drying method and the resulting dry powder.

According to a further advantageous embodiment, the spray drying is performed using an atomizing gas that is fed to the atomizing nozzle, preferably nitrogen, and a heated carrier gas, preferably dry air, that is fed to a spray drying chamber of the spray drying apparatus. Those gasses can be filtered for dust and particle removal prior to feeding them to the atomizing nozzle or spray drying chamber, respectively.

In a further embodiment of the invention an additional drying step is carried out, in which a carrier gas is fed through the spray drying apparatus, especially the atomizing nozzle, without being mixed with aqueous solution or suspension and preferably while the spray dried powder has already been transferred to the accumulation means. Although it is advantageous to provide the carrier gas to the spray drying apparatus in the same way as during the actual spray drying process, because the process and apparatus do not need to be adapted to perform the respective process step, the carrier gas for the additional drying step can also be fed through the spray dried powder by different means, for example a separate nozzle or the like.

The additional drying step or the additional spray drying step reduces the residual moisture without harming the polyplexes and/or the siRNA and thereby increases the fine particle fraction (FPF) of the powder. This again is advantageous for the delivery to cells and the resuspension of the polyplexes.

In another preferred embodiment, the additional drying step is carried out with the temperature in the vicinity of the outlet opening being controlled by temperature controlling means to be set to an upper threshold temperature, in particular an upper threshold temperature of which is equal to or below a melting temperature of naked siRNA, especially within a range of 5°C, in particular in the range of 3°C, below the melting temperature of the respective naked siRNA. In other words, the additional drying step can be carried out at the same temperature as the spray drying itself. This is especially advantageous as this reduces the amount of process variables or method parameters of the spray drying method. Thus, the additional drying step can be carried out by keeping the carrier gas running for an additional time period after the aqueous suspension has been fed through the atomizing nozzle, in particular without changing the carrier gas temperature. In a preferred embodiment the volume flow of the carrier gas also remains unchanged during the additional drying step. The additional drying step may take place directly after spray drying the aqueous suspension containing the siRNA. Alternatively the spray drying may also comprise a method step, in which after the spray drying of the aqueous suspension an intermediate solution or suspension, such as RNase free water is performed, in particular for a time period of 1 to 5 minutes, before the additional drying step is performed.

In an advantageous embodiment the additional drying step is performed for 3 to 10 minutes, in particular 4 to 6 minutes, especially for 1 to 1.5 minutes per 10µg siRNA. For the additional drying step, as for the spray drying itself the carrier gas can be fed to the spray drying apparatus with a flow rate of about 460 to 480 NL/h (standard litre per hour). Alternatively, the flow rate can be set to 13 to 17 NL/h per µg siRNA, in particular 14 to 16 NL/h per µg siRNA, for the spray drying and/or the additional drying step.

It is further preferred if the additional drying step is carried out until a residual moisture of less than 3%, in particular less than 2% is reached. This leads to a longer shelf life of the resulting dry powder.

It was further found that the siRNA was preserved in a bioactive state to a very high degree during the additional drying step, when polyethyleneimine-graft-polycaprolactone-block-polyethylene glycol (PEI-g-PCL-b-PEG or PPP) and/or a bioconjugate of polyethylenimine with peptides or proteins such as Transferrin conjugated polyethylenimine (Tf-PEI) was used as polyamine. This is especially advantageous, because the Tf-PEI is a polyamide that is very promising to target T-Cells with the respective dry powder. PPP is advantageous due to its low cytotoxicity.

The above aim is also achieved by a powder containing bioactive siRNA in the form of polyplexes, in particular polyelectrolyte complexes, formed from at least a polyamine and/or polyamide and/or polyester and siRNA, wherein the polyplexes are encapsulated into water soluble excipients, in particular mannitol and/or trehalose, preferably with aerodynamic diameters (MMAD) between 0,5 and 10µm, in particular 1 and 5µm, wherein the powder is produced according to the method described above.

According to a preferred embodiment of the invention, the powder can be resuspended in water to polyplexes or polyplex suspensions which are not significantly different, in particular which do not vary more than +/- 10%, from their initial formulation before spray drying in regard to size, polydispersity and zeta potential. This embodiment allows for an efficient uptake of the powder into cells. Thus, the inventive powder does not show significant agglomerates after resuspension that would lower the percentage of polyplexes that can potentially be absorbed by cells.

The inventive powder allows a long shelf life due to the low residual moisture. Moreover, the powder comprises a high amount or high yield of the initially provided siRNA in a bioactive state, thereby allowing an effective production of siRNA powder. Since the bioactive siRNA content is reproducible, the powder is an excellent platform for pharmaceutical registration process for nucleic acid based drugs.

According to a preferred embodiment the polyamine of the encapsulated polyplexes is polyethyleneimine-graft-polycaprolactone-block-polyethylene glycol (PEI-g-PCL-b-PEG or PPP) and/or bioconjugates of polyethyleneimine or polyspermine with peptides or proteins such as Transferrin conjugated polyethylenimine (Tf-PEI). Those polyamines are promising candidates for a pharmaceutical use of the dry powder.

In a preferred embodiment the particles exhibit a spherical structure.

The above aim is also achieved by a use of a powder composition or blend comprising at least a powder disclosed above as a pharmaceutical dosage form, in particular for pulmonary delivery. In this respect it is especially advantageous that the spray drying process results in a particle size and size distribution which is suitable for pulmonary delivery.

According to a preferred embodiment of the inventive use, the siRNA is active in silencing the translation of messenger RNA into proteins causing lung diseases.

Inventive and comparative non-inventive embodiments will be described in more detail below. The inventive embodiments are only special examples of the technical disclosure:

Materials and Methods

Double stranded siRNA targeting green fluorescent protein (DsiRNA EGFP 1) (siGFP) and scrambled nonspecific control (siNC) are available from IDT (Integrated DNA Technologies, Inc., Leuven, Belgium). The invention will be described in more detail with respect to those siRNA specimens. However, the invention is not limited to the use of such siRNA. Other siRNA may be used, especially for pharmaceutical uses.

Hyper branched polyethylenimine (PEI) (25kDa) is sold by BASF (Ludwigshafen, Germany). Hetero-bifunctional polyethylene glycol (HO-PEG-COOH, 2.5 kDa) is produced by JenKem Technologies (Plano, TX, USA). ε-Caprolactone, Heparin from porcine intestinal mucosa (H3393, >180units/mg, grade I-A), picrylsulfonic acid (TNBS) (P2297), TRIS EDTA Buffer Solution 1x (TE-buffer) (93283), TRIS EDTA Buffer Solution 100x for NGI analysis (T9285), tris borate EDTA buffer (TBE-buffer) (T 3913), RPMI-1640 Medium (R8758), fetal bovine serum (FBS) (F9665), Penicillin-Streptomycin (P/S) (P4333), G 418 disulfate salt solution (G8168), Dulbecco's Phosphate Buffered Saline (PBS) (D8537) and D-Mannitol are available through Merck KGaA (Darmstadt, Germany). D(+)-Trehalose dihydrate (28719.290) was acquired from VWR International GmbH (Darmstadt, Germany). Black and white 96 well plates (10307451), GeneRuler Ultra Low Range DNA Ladder (10400280), SYBR™ Safe™ DNA-Gel staining and polyacrylamide gels (Novex™ TBE Gels, 4-20%, EC62252BOX) are sold by Fisher Scientific (Schwerte, Germany). SYBR™ Gold dye was obtained from Life Technologies (Carlsbad, CA, U.S.A.). Pumpsil® tubings from Watson-Marlow GmbH (Rommerskirchen, Germany) were used and had an inner diameter and a thickness of 1.6 mm.

### Polyamine synthesis

Synthesis of the polyethyleneimine-graft-polycaprolactone-block-polyethylene glycol (PEI-g-PCL-b-PEG or PPP) polyamine can be performed as previously described. [24] This document as well as EP 3 277 743 A4 and WO 2008 151 150 A2 are incorporated by reference as part of the current disclosure. In brief, the triblock copolymer of polyethyleneimine-*graft*-polycaprolactone-*block*-polyethylene glycol (PEI-g-PCL-b-PEG) (PPP) was synthesized by coupling the heterobifunctional diblock copolymer acrylated-PCL-b-PEG-alkyne to PEI. Characterization was performed by $^1$HNMR and UV Spectroscopy as described before [10].

The Transferrin conjugated polyethylenimine (Tf-PEI) was synthesized in accordance with the disclosure of [24a] as well as US 2002 013 767 0 A1, which are also incorporated herein by reference.

Other possible polyamines can be incorporated into poly(urethanes), poly(ureas), poly(acrylamides), poly(amides), poly(esters) or poly(aminoesters). Spermine is a biogenic polyamine, consisting of two primary and two secondary amines, found as a polycation at all pH values. Spermine-based polyacrylamides can be synthesized via free radical or living radical polymerization (e.g. RAFT) of the (meth)acrylate of an active ester of acrylic acid and post-polymerization with a spermine-species or directly via polymerization of an acrylamide consisting of spermine. Such polyamines are preferred, since they are biodegradable and biocompatible.

### Polyplex preparation

Stock solutions of siGFP and PPP were prepared with a concentration of 100 $\mu$M and 1 mg/ml, respectively. Polyplexes were prepared with a total amount of 30 $\mu$g of siGFP. Therefore, the amount of PPP ($m_{PEI}$) in $\mu$g was calculated as follows:

$$m_{PPP} = \left( \frac{m_{siGFP}}{17950,36 \; g/mol} \right) \cdot 43.1 \cdot N/P \; (Eq.1)$$

The calculated amount of PPP was diluted up to 250 $\mu$L in a specified solvent (highly purified water (HPW), trehalose or mannitol 5 or 10%) and 250 $\mu$L of the same solvent containing 30 $\mu$g siGFP was added. To allow polyplex formation, the mixture was incubated for 10 minutes. Then, 4500 $\mu$L of the same specified solvent was added and the polyplex suspension was incubated for another 10 minutes.

### Adsorption to tubing material

In order to reduce adsorption of polyplexes by tubing material during pumping through the respective tubing, different tubing materials were washed prior to experiments with pre-heated HPW and allowed to dry. After insertion into the Masterflex L/S (7520-47, Cole-Parmer GmbH, Wertheim, Germany), equipped with the Easy-Load II head module (77201-60), a pump rate of 1.2 ml/min was set. Polyplexes were prepared in HPW and pumped through the tubing and collected in a 5 ml tube for further analysis. This procedure was carried out in triplicates.

### Spray drying

For microparticle preparation a B-290 (Büchi Labortechnik, Essen, Germany) was used as spray drying apparatus. As Pumpsil® tubings did not fit into the Büchi pre-installed pump, the Masterflex L/S (see above) was used with a pump rate of 1.2 ml/min. Nitrogen was used as atomizing gas, whereas carrier gas was air. In order to avoid dust and other airborne particles, both nitrogen and air supply were filtered through a 0.22 $\mu$m pore filter. To ensure sufficient heating of the air supply and to avoid overheating of the Büchi vacuum pump, pressurized air was used. The aspirator was set to 70% and vacuum was set to -42 mbar by adjusting the level of pressurized air. The airflow was set to 40 mm corresponding to 473 NL/h. For particle collection, a high efficiency cyclone was attached to an outlet opening of the spray drying apparatus. Polyplexes were prepared in 5 or 10% m/V trehalose or mannitol.

All formulations were prepared the same day to avoid inter-day differences related to ambient temperature and humidity, for example. For analysis three batches were produced on three different days. In the case of different inlet-temperatures (T-In), the measured outlet-temperatures (T-Out) were indicated next to T-In for a better understanding of the process. During the spray drying process, minor changes of T-Out were observed. Hence, T-Out was reported as mean with a deviation of $\pm$ 1.5° C.

Additional Drying Step

For one spry drying run, a total amount of 30.00 μg siGFP is diluted with a 5% trehalose solution in RNAse free water to a total volume of 250 μl. Polyplexes are prepared at N/P ratio of 10. Therefore, the required amount of PPP [1] is also diluted with a 5% trehalose solution in RNase free water to a volume of 250 μl. The PPP solution is added to the siGFP solution and pipetted vigorously up and down. It is incubated for 20 minutes for polyplexes formation. Subsequently, 4,500 μl of 5% trehalose solution are added to the polyplex suspension. The tube is carefully turned to mix. In the meantime, the spray dryer is turned on; the parameters are set at 145°C inlet temperature, 70% aspirator, an airflow of 40 mm (473 NL/h) and water cooling of the nozzle. RNase free water is spray dried at 1.4 mL/min. If a constant inlet temperature of 145°C is reached, the spray drying process with the polyplex-trehalose dispersion begins. At the end of the process, the sample tube is replaced with RNase free water which is spray dried for another 2 minutes. Hereinafter, the pump is stopped. The spray drying process is finished, and the additional spray drying step of the nano-embedded microparticles starts. A timer is set to five minutes and all other parameters are kept constant. Therefore, the subsequent drying step takes place at 145°C inlet temperature. Finally, the spray drying process is stopped, and the spray dried powder collected.

After the spry drying process, all powder is collected, weighed, and aliquots are used for further analysis. To quantify the PPP and siGFP content within the microparticle powder following spray drying, an aliquot of the powder was dissolved in HPW (Highly purified water), and heparin competition assays were performed to disassociate the siGFP-PPP polyplexes within the NIM powder. Subsequently, the siGFP that was released from the polyplexes was determined by a modified SYBR® gold assay [1] while the PPP concentration was determined by TNBS assay [2].

First, the batch of NIM powder was dissolved in 2 mL HPW in a volumetric flask. Following procedure was executed in triplicates: According to the theoretical content of siGFP, a volume was chosen to achieve approximately 0.04 μg of siGFP per sample. This solution was diluted to a final volume of 150 μL per sample with HPW in a tube. Afterwards, 75 μL of a 58 k IU heparin solution was added to each tube to achieve complete disassociation of polyplexes after 2 h. This solution was further diluted to 450 μL and distributed into a white 96 well plate (Thermo Scientific™ BioLite microwell plate, Thermo Scientific GmbH, Schwerte, Germany) in triplicates of 100 μL each. For quantification of siGFP, 30 μL of a 4 × SYBR® gold solution was added to each well and incubated for 10 min in the dark at room temperature. The fluorescence of each sample was quantified using a Synergy 2 multi-mode microplate reader (BioTek Instrument, Winooski, VT, U.S.A.) at excitation wavelength of 485/20 nm and emission wavelength of 520/20 nm and compared to a freshly prepared standard curve of free siGFP incubated with SYBR® gold dye.

For quantification of PPP, 100 μL of each batch dissolved solution in 2 mL borax buffer 0.1 M (Sodium Borax Decahydrate, Sigma Aldrich, Germany) was incubated with 30 μL of 3 mM TNBS solution in triplicates and absorbance at 405 nm was determined after a 1 h incubation using a Synergy 2 multi-mode microplate reader (BioTek Instrument, Winooski, VT, U.S.A.). The measured absorbance was compared to a freshly prepared standard curve of free PPP incubated with TNBS reagent. To verify each quantification assay, internal standards were prepared freshly and analyzed in parallel. These standards consisted of siGFP and PPP of known amounts which were chosen according to the theoretical amount of siGFP or PPP being analyzed in the sample, respectively. These were 0.04 μg siGFP per well for nucleic acid quantification and 0.48 to 1.2 μg PPP for polymer quantification. Measurements with deviations of less than 10% of the internal standard were considered as precise and taken into account for further analysis.

The residual water content of the NIM powder after spray drying was determined by coulometric measurement using an Aqua 40.00 Karl Fischer Titrator with corresponding software from Analytik Jena AG (Jena, Germany). The samples (approx. 15 mg) were loaded into 2R vials, placed into the heating chamber and measured at 100 °C until the measurement drift reached the start drift ≤+2 μg/min or until a final measurement time of 10 min. The start drift was established after approximately 1 h of equilibration showing a rate of less than 10 μg/min. Hydranal Coulomat AG (Riedel-deHaën, Seelze, Germany) was used as reagent. Before each session, the titrator was calibrated with a 1% water standard. The moisture content was calculated as the % weight of water relative to the overall sample weight.

Z-Averaqe and PDI measurements

To compare the effects of pumping and spray drying on polyplex content, 70 μL of freshly prepared polyplex suspension was compared to polyplexes after further processing. For pump adsorption experiments, 70 μL were taken after pumping. For redispersability of spray dried formulations, approximately 3.5 mg and 7.0 mg for 5% and 10% matrix formulations, respectively, were dissolved in 70 μL HPW. All samples were analyzed in disposable cuvettes (Brand GmbH, Wertheim, Germany) and analyzed with the Zetasizer Nano ZS (Malvern Instruments Inc., Malvern, U.K.). Therefore, the refractive index of water, mannitol or trehalose at 25°C for the indicated concentrations were set in the software, and detection was performed with the backscatter angle of 173°. For each experiment, measurements were taken in triplicates with 15 runs each and averaged afterwards.

Static Light Scattering

A few μg of spray dried powder was suspended in about 2 ml of diethyl ether. About 30 minutes prior to measurements the HORIBA LA-950 (Retsch Technology GmbH, Haan, Germany) was switched on for equilibration. The cuvette was filled with diethyl ether, inserted into the device and acquisition and blank measurements were recorded. The sample suspension was mixed thoroughly by pipetting up and down and small amounts were added into the cuvette. Additionally, a small magnetic stir bar was inserted for sample stirring. The speed was adjusted to achieve light transmittance between 85 and 90% of red light and between 80 and 90% of blue light. The amount of powder in the cuvette was adjusted accordingly. Following indices were used for measurements:

| | |
|---|---|
| Real refractive index - mannitol: 1.330 | Imaginary index - mannitol: 10.0 |
| Real refractive index - trehalose: 1.652 | Imaginary index - trehalose: 2.0 |
| Refractive index diethyl ether: | 1.352 |

Imaginary indices were chosen experimentally to obtain smallest possible R parameter (here R<0.08 at all measurements). The quality of these measurement is given by the R parameter which decreases if the predicted scattering of the particle size distribution measurements fits with the detected scattering of the sample. [25] Measurements were executed on three different batches.

Scanning Electron Microscopy

A small amount of powder was placed on top of a stub covered with double-sided carbon tape. Before analysis, the stub was coated with carbon under vacuum for 40 s. The morphology of particles was examined by scanning electron microscopy (SEM) using a FEI Helios G3 UC (Thermo Fisher Scientific, Schwerte, Germany).

Residual water content

For trehalose and mannitol microparticles, approximately 5 mg and 15 mg were weighed into a 2R vial, respectively. Three different batches were measured in triplicates, each. Also, a 1% water standard was prepared with approximately 10 mg. After filling, a small piece of ceramic wool (Analytik Jena AG, Jena, Germany) was applied on top to avoid particle suction through the titrator. Vials were closed with a plastic stopper. Empty vials acting as blank values were treated accordingly. For coulometric measurements an Aqua 40.00 Karl Fischer Titrator with corresponding software from Analytik Jena AG (Jena, Germany) was used. First the oven was heated to 100°C and the system was cleared of residual humidity by inserting an empty closed vial and activating the pump until a final drift of less than 8.0 μg/min was reached. The specified drift was used and stop conditions were set to a total measurement time of 10 minutes or until the drift reached ≤2.0 μg/min of the initial drift. Blank measurements were executed and automatically subtracted from standard and samples.
Measurements were considered correct if the 1% water standard measurement resulted in a value between 0.9 and 1.1 %.

Differential Scanning Calorimetry

For calorimetric measurements 3 to 5 mg of sample were weighed into a concavus pan and closed. The reference was an empty closed concavus pan. Reference and sample were inserted into the oven at a set point of 25°C and the oven was closed. Measurements were taken with a DSC 214 Polyma (Erich NETZSCH GmbH & Co. Holding KG, Selb, Germany) starting from -10 °C with a ramp of 8° K/min until temperature reached 160°C for trehalose or 200°C for mannitol formulations. Data was analyzed using the Proteus Analysis software.

XRPD

For identification of crystalline and or amorphous structures XRPD was executed with a 3,000 TT diffractometer (Seifert, Ahrensburg, Germany). Equipped with a copper anode with a voltage of 40 kV and a current of 30 mA, a wavelength of 0.154178 nm was used. The voltage of the scintillation detector was 1,000 V. Samples placed on the copper sample holder were analyzed in the range of 5-40° 2-theta in steps of 0.05° 2-theta.

Aerodynamic properties

For analysis of aerodynamic properties apparatus E of the European Pharmacopoeia was used from Copley Scientific (Nottingham, UK). The next generation impactor (NGI) was fitted with a preseparator (PS) and an induction port (IP). The instrument was connected to a critical flow controller (TPK 2, ERWEKA GmbH, Langen, Germany) to ensure correct valve opening for a predetermined time to allow a total volume of 4 L air passing through the instrument for each measurement. Further on, the TPK was connected to a high performance vacuum pump (HVP 1000, ERWEKA GmbH) generating a flow rate which was set to 30 L/min (volumetric L/min) by a TSI 4040 flowmeter (TSI Instruments Ltd., High Wycombe, UK). A stock of 0.167x Tris-EDTA (TE) solution was prepared by mixing 0.5 ml 100x TE with 299.5 ml HPW. Prior to each analysis, the preseparator was filled with 10 ml of Heparin-TE solution (23.3 mg Heparin in 60 ml 0.167x TE) (HTE) and cups were coated with 10 $\mu$L of a solution containing 83% glycerin, 14% ethanol and 3% Brij 35. [26] Cotton swabs pre wetted with coating solution were used to distribute the coating solution across the entire cup area. For analysis 4 or 8 hydroxypropylmethylcellulose capsules were loaded with approximately 45 mg of 5% or 10% m/V spray dried formulations, respectively. Each capsule was loaded into a Handihaler® (Boehringer Ingelheim Pharma GmbH & Co. KG, Ingelheim, Germany) and activated by piercing. According to the manufacturer's manual, the capsules were discharged twice with an interval between the two actuations of 5 s. After discharging the content of capsules into the impactor, the IP was carefully taken off and closed with two rubber stoppers after 10 ml of HTE were inserted. Also, the PS was carefully removed and both openings were closed with plastic stoppers. Both, IP and PS, were shaken vertically and horizontally for 1 minute. Finally, the NGI was disassembled and the small cups were filled with 2 ml HTE whereas the greater cups were filled with 4 ml HTE. All cups were covered with a plastic lid to avoid solvent evaporation. The cups were placed on a shaker for 5 to 10 minutes and the rotation speed was set in a fashion to avoid spilling but ensure complete dispersion of particles. Three aliquots of 100 $\mu$L from each stage including IP and PS were prepared for further analysis. The mass of siGFP deposited on each stage was analyzed as described under 2.12 with an extended standard point line towards the lower limit. This experiment was carried out with three different batches. The mass median aerodynamic diameter (MMAD), geometric standard deviation (GSD), fine particle dose (FPD) and fine particle fraction (FPF) were calculated as described in the European Pharmacopoeia [19]. 'Fine particles' were considered all particles below 5 $\mu$m.

siRNA and PPP quantification

Quantification assays were performed as described earlier. [12] In short, 50 mg for 5% and 100 mg for 10% matrix formulations, respectively, were transferred into a 2 ml volumetric flask and dissolved in HPW to release polyplexes. These solutions were used for the following assays:

*TNBS assay*

An aliquot of 100 $\mu$L of each sample was taken and mixed with 0.088% m/v TNBS in 0.1 M borax. After an incubation time of 1h, samples were analyzed with a quartz cuvette in a UV-1600PC spectrophotometer (VWR International GmbH, Darmstadt, Germany) at an absorbance of 405 nm. Results were compared with an equally treated standard dilution series (0.166 $\mu$g - 1.914 $\mu$g) where a corresponding amount of siGFP was added to avoid biases. Measurements were only considered for further analysis if an internal standard (iS), prepared as described in 2.3., showed a deviation of less than $\pm$ 10 % compared to the theoretical amount.

*Heparin SYBR gold assay (HeoSYBR)*

An aliquot of 60 $\mu$L of each sample was taken and diluted with HPW to 150 $\mu$L. To dissociate siGFP from PPP, 75 $\mu$L of 2.33 mg/ml heparin solution in TE buffer was added and incubated for 2h. After dilution with HPW to 450 $\mu$L, triplicates of 100 $\mu$L were pipetted into a black 96 well plate. A dilution series starting at 0.09 pg/100 $\mu$L was prepared and added in triplicates into the same 96 black well plate. To verify full dissociation of siGFP and PPP, an iS was prepared as described in 2.3, treated alike and analyzed in triplicates. A 4x SYBR gold solution in HPW was prepared for intercalation of double stranded RNA and 30 $\mu$L were added to each well with an 8-channel multi pipette. Fluorescence was measured at an excitation wavelength of 485/20 nm and an emission wavelength of 520/20 nm on a FLUOstar® Omega multimode microplate reader (BMG LABTECH GmbH, Ortenberg, Germany). Measurements were considered for further analysis if iS showed a deviation of less than $\pm$ 10 % compared to the theoretical amount.

Integrity test

To achieve a final gel loading amount of 150 ng siGFP per lane (m(siGFP)$_{lane}$), siRNA losses detected by HepSYBR were considered (recovery) and the following calculation was used to determine the amount of spray dried powder (m(NIM)):

$$m(NIM) = m(siGFP)lane * \frac{m(tsc)}{m(siGFP)sd} * recovery \text{ (Eq.2)}$$

where m(tsc) is the total solid content of the spray dried powder and m(siGFP)$_{sd}$ is the initial amount of siGFP used for spray drying i.e. 30 μg. Powder was weighed and reconstituted in 15 μL of HPW and 5 μL of Heparin (12 μg Heparin / 5 μL TE-buffer). After 30 minutes of incubation, 4 μL of the 6x loading dye was added and a 4-20 % TBE gel (EC62252BOX, ThermoScientific, Germany) was loaded with 24 μL of each sample. For control, free siGFP and siGFP with heparin were loaded as well. The gel was run at a constant voltage of 200 V for up to 1 hour in Tris-Borate-EDTA buffer (TBE) until lanes separated. Gels were taken off the chamber and placed in 20 ml of a 1x SYBRsafe solution for 30 minutes under 50 rpm shaking. Gels were analyzed using a ChemiDoc fluorescence detector (Bio-Rad Laboratories GmbH, Feldkirchen, Germany).

*In vitro* knockdown

For *in vitro* knockdown performance a non-small cell lung cancer line H1299 (ATCC CRL-5803) stably expressing enhanced green fluorescence protein (GFP) was used. Cells were cultivated in RPMI-1640 supplemented with FBS (10%), P/S (1%) and G418 (0.4%) for selection at 37°C with 5% $CO_2$. Cells were seeded into 24 well plates with a density of $2 \times 10^5$ cells per well and a total volume of 500 μL medium. On the day of transfection, the medium was replaced by 400 μL fresh medium and 100 μL of samples were applied to obtain a final concentration of 100 nM siGFP or siNC. As different spray dried formulations at different concentrations were tested and taking detected losses into account, the amount of powder and hence of excipient had to be adjusted. Therefore, samples within the same group were treated to contain equal amounts of excipient for better comparability. After 72h, medium was discarded, cells were washed with PBS, trypsinized and collected. After centrifugation at 400 rcf for 5 minutes, supernatant was discarded and the cell pellet was resuspended in PBS. Samples were analyzed by flow cytometry (Attune® Acoustic Focusing Cytometer, Life Technologies) and the median fluorescence intensity (MFI) was measured using 488 nm excitation and a 530/30 nm band pass emission filter set (BL-1H). Samples were run in triplicates for each batch, with each sample gated by morphology based on forward/sideward scattering for a set of 10,000 viable cells. Triplicates of each batch were summarized by generating the mean value.

Tf-PEI and GATA3 formulation

Transferrin-PEI (Tf-PEI) was prepared as described in [27, 28]. To downregulate GATA3, two different siRNA sequences targeting GATA3 (siGATA3) were used from QIAGEN GmbH (Hilden, Germany) in a mixture of 1:1 (HS_GATA3_8 - SI04212446, and HS_GATA3_9 - SI04364101).
The formulation was spray dried as described in chapter 2.4. containing 1760 pmol/5 ml of 5% v/v trehalose or mannitol. The powder was analyzed according to chapter 2.11. to ensure exact amounts of siGATA3 for transfection.
Transfection was executed with primary CD4+ T cells which were isolated from freshly obtained buffy coats (DRK, Berlin, Germany). Cells were cultured in RPMI medium supplemented with 10% FBS, 1% P/S, 10 mM HEPES, 1 mM sodium pyruvate and 4500 mg/L glucose.
For GATA3 knockdown $8 \times 10^6$ primary T cells were seeded in a 48 well plate containing 200 μL medium. Primary T cell activation was executed by using Dynabeads™ Human T-Activator CD3/CD28 (11131D, Life Technologies) following the supplier's protocol of mixing beads and cells 1:1. Spray dried powder were redispersed in nuclease free water. Controls consisting of siGATA3 or siNC were prepared with Tf-PEI or LF. After two days of T cell activation samples were applied to cells thereby achieving a final concentration of 100 nM siRNA. Cells were incubated for 48h and after removal of Dynabeads lysed with the PureLink RNA mini kit according to the manufacturer's protocol (12183025, Thermo Fisher Scientific). In short, cells were washed, lysed, and RNA was isolated with an additional DNase digestion step. Afterwads, cDNA was synthesized using the high capacity cDNA Synthesis kit (#4368814, Applied Biosystems). After obtaining cDNA, the solution was diluted 1:10 and a qRT-PCR was run with custom synthesized GATA3 forward and reverse primers (Thermo Fisher) and β-actin primers (Qiagen, Hilden, Germany) for normalization. Cycle thresholds were acquired by auto setting within the qPCRsoft software (Analytik Jena AG, Jena, Germany).
Advantageous embodiments and comparative embodiments are discussed below with reference to the Figures, which show:

Fig. 1A:     Quantification of siGFP after spray drying of polylexes (N/P 5) at various T-In/T-Out;

Fig. 1B: Agarose gel of redispersed polyplexes after spray drying with indicated T-In. siGFP was released from polyplexes upon incubation with heparin. Lane 1: internal standard (iS), Lane 2: powder spray dried at 65° T-In, Lane 3: powder spray dried at 145° T-In Lane 5: low range DNA base pair ladder;

Fig. 1C: Quantification of siGFP after pumping polyplexes through standard silicon tubing and Pumpsil tubing. (n=3);

Fig. 2: DLS measurements of freshly prepared (dark grey bars) and redispersed (light grey bars) polyplexes. PDI is indicated by red circles. (n=3);

Fig. 3: Agarose gel of redispersed polyplexes after spray drying with mannitol or trehalose at indicated T-In. siGFP was released from polyplexes upon incubation with heparin. M5/T5: 5% m/V mannitol/trehalose formulation of siGFP-PPP; spray dried. M10/T10: 10% m/V mannitol/trehalose formulation of siGFP-PPP; spray dried. L: Ultra low range base pair ladder;

Fig.4: Quantification of A) siRNA (siGFP) and B) polymer (PPP) after spray drying at two different temperatures with two different excipients at two different concentrations. (n=4) M5/T5: 5% m/V mannitol/trehalose formulation of siGFP-PPP; spray dried. M10/T10: 10% m/V mannitol/trehalose formulation of siGFP-PPP; spray dried;

Fig. 5A: DSC measurements of Trehalose formulations: 1.2) T5-120°, 2.2) T5-145°, 3.2) T10-120°, 4.2) T10-145°, 5.2) trehalose dehydrate;

Fig. 5B: DSC measurements of Mannitol formulations: 1.2) cryst. Mannitol, 2.2) T5-120°, 3.2) T5-145°, 4.2) T10-120°, 5.2) T10-145°;

Fig.6A: XRPD measurements of Trehalose formulations spray dried at 145°/79° T-In/T-Out;

Fig. 6B: XRPD measurements of Mannitol formulations spray dried at 145°/79° T-In/T-Out;

Fig. 7: Particle size distribution as measured by SLS of M5-145°, M10-145°, T5-145°, T10-145°;

Fig. 8A-D: SEM pictures of A) T5-145° B) T10-145° C) M5-145° D) M10-145°. White arrows mark water bridges between single trehalose particles;

Fig. 9: *In vitro* knockdown of GFP within a H1299 cell line stably expressing eGFP. Within each group, samples contain the same amount of excipient as indicated by the legend. LF: Lipofectamine, PPP: PEG-PCL-PEI block copolymer, siNC: negative control siRNA, siGFP: siRNA sequence against eGFP, SD: spray dried polyplexes consisting of PPP and siGFP at 5% (w/V) total solid content at 145°C T-In;

Fig. 10: *Ex vivo* knockdown of GATA3 within primary CD4+ T cells; LF: Lipofectamine, Tf-PEI: Transferrin conjugated PEI, siNC: negative control siRNA, siGATA: siRNA sequence against GATA3, M5-145°/T5-145°: spray dried polyplexes of Tf-PEI and siGATA at 145°C T-In with 5% Mannitol or 5% Trehalose;

Fig. 11: Spray drying of nanoparticles consisting of siGFP and PEG-PCL-PEI (PPP) in 5% trehalose solution (A) and the subsequent drying process of the nano-embedded microparticles (NIMs) at the same temperature for 5 minutes (B). The spray drying parameters were set to 145 °C for the inlet temperature (T-In), an aspirator performance of 70% and an airflow of 40 mm (473 NL/h). The pump flow was set to 1.4 mL/min and stopped for the subsequent drying;

Fig. 12: siGFP- and PPP-loss after subsequent drying of spray dried nanoparticles in 5% trehalose solution. The subsequent drying time was five minutes. siGFP-loss was detected by HepSYBR assay and PPP-loss by TNBS assay. Data points indicate mean ± SD. (n=4),

Fig: 13: Residual moisture of spray dried nanoparticles before and after subsequent drying. Karl-Fischer characterization of spray dried siGFP-PPP nanoparticles in 5% trehalose before and after subsequent drying (n=4). One-way ANOVA, Tukey post-test, **P < 0.05.

Heat evaluation and integrity

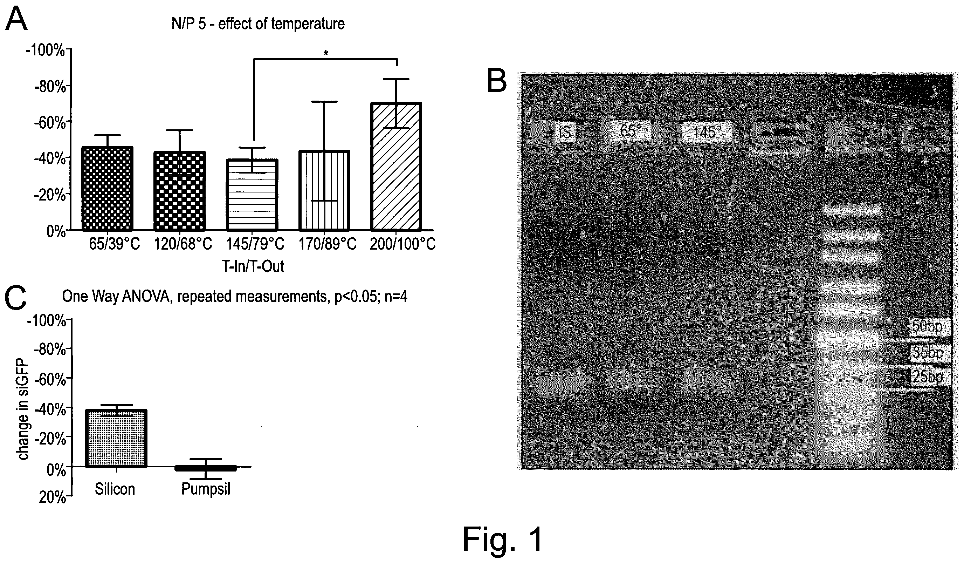

Spray drying is a well-known technique for preparing microparticles. However, heat is a crutial parameter which could have a tremendous effect on siRNA. Hence, we spray dried polyplexes in presence with trehalose (10% m/V) at various inlet-temperatures (T-In), which resulted in respective temperatures (T-Out) in the vicinity of the outlet opening. Figure 1 shows three inventive examples with T-Out below 90°C and a comparative example with T-Out being 100°C. As shown in Figure 1, increasing T-In up to 170° and the respective T-Out of 89°C had no significant effect on the quantity of siGFP. At 200°C/100°C T-In/T-Out, a significant increase in siGFP loss was detected.

As naked siRNA melts at around 90°C and switches from the double stranded to the single stranded form, it was found according to the invention that the formation of polyplexes and their incorporation into the matrices of the excipients did not thermally or chemically shield or stabilize the siRNA significantly making it less heat sensitive and allowing higher spray drying temperatures, especially T-Out, compared to the respective naked siRNA. Thus, it is an important achievement of the invention that successful spray drying with minimized losses of intact siRNA includes avoiding heating the siRNA formulation to temperature above the melting temperature of naked siRNA for extended periods of time.

The product temperature reached during the spray drying process is determined as the T-Out. [29] Hence, the temperature which affects the product is equal to T-Out and therefore crucial for stability and integrity. For T-In at 200°C and a subsequent T-Out of 100°C, melting of siRNA seems to have occurred and consequently resulted in higher susceptibility to degradation. [22, 30] Such high temperatures can potentially also lead to polyplex dissociation and siRNA release from the polyplexes. Protection of siRNA against heat and shear forces might therefore be hindered which could explain the greater loss at such elevated temperatures. However, the loss of siRNA is restricted as the exposure time of polyplexes to these temperatures is extremely low. Also, a large variation in siRNA recovery, reflected in a comparably great standard deviation, was observed for spray conditions at 170°/89°C T-In/T-Out. Here, T-Out with a small deviation (89° $\pm$ 1.5° C) just reached the melting temperature of siRNA. Again, this could induce melting in some cases, leading to greater siRNA losses. Therefore, the temperature which can be used for the preparation of siRNA via spray drying with an advantageous minimal loss of siRNA in our case is determined at 145°C T-In and a subsequent T-Out of 79°C. Furthermore, it seems possible that spray drying can also be successful without elevated losses at even higher T-In if the equipment is set up and the method is carried out in a fashion that T-Out remains below 90°C. This could be achieved for example by increasing the feeding or aspirator rate when increasing T-In at the same time. [29]

As the siRNA quantification described here relies on intercalation of a fluorescent dye which does not reflect the nucleic acid integrity, the latter has to be confirmed separately. This was achieved via gel retardation assay with samples obtained at the lowest T-In and the highest acceptable T-In (145°C). Figure 1B confirms the duplex length of about 25 bp for the internal standard as well as the spray dried samples obtained at both process parameters. Hence, siRNA integrity in the recovered material even at T-Out of 79°C was maintained.

Although T-In/T-Out were reduced to minimize siRNA losses and duplex integrity was confirmed, nucleic acid losses were still rather high with approximately 40%. To further elucidate the reasons for these losses the effect of pumping polyplex suspensions from the sample container into the spray dryer was investigated. To test this effect, a polyplex suspension was pumped through the silicon tubing connected to the spray dryer and was collected afterwards. Interestingly, quantification of siGFP after pumping with regular quality silicon tubing, which was used in the aforementioned experiments, revealed losses of around 40% (Figure 1C).

These losses correspond to detected losses of redispersed polyplexes after spray drying. We therefore inferred that the measured losses during spray drying (Figure 1A) are not solemnly linked to the spray drying process itself but also depend on the pumping step and the used tubing material.

It is believed that the source of adsorption of siGFP is due to hydrophobic interactions which were previously only researched for DNA. [15] Furthermore, we tested whether high quality silicon tubing would reduce siRNA adsorption. And indeed, siGFP losses were reduced to 0% when using Pumpsil tubing (Figure 1C). One explanation for the different adsorption behavior of siRNA polyplexes on high vs. regular quality silicon tubings might be abrasion which takes place in the latter tubing and results in cavities. These superficial changes lead to an increase and regeneration of surface area and hence to an increase of possible interactions between siGFP and the tubing material. Therefore, low abrasion tubings such as Pumpsil seem advantageous for the processing of polyplexes. The detailed mechanism for adsorption however is not fully understood yet.

Nanoparticle Characteristics

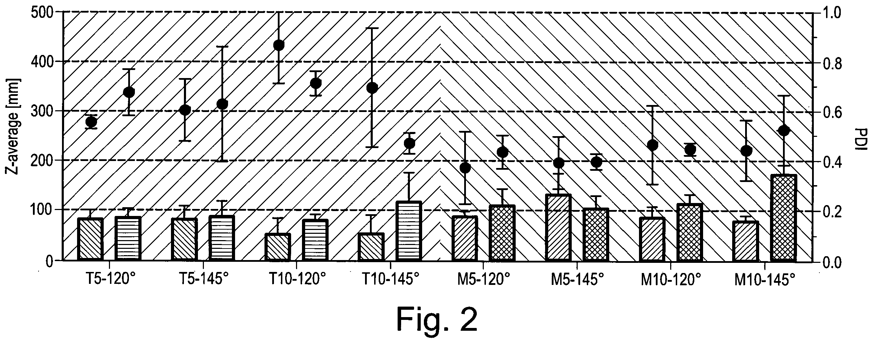

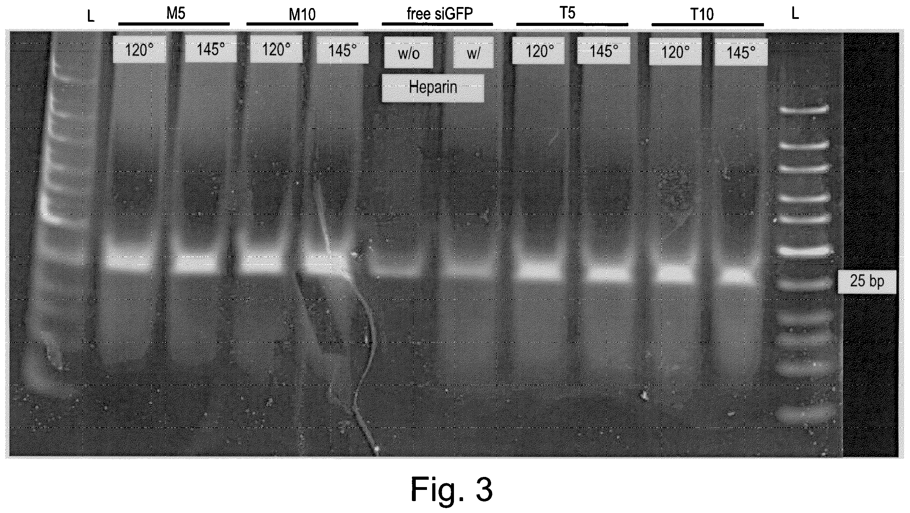

Besides heat, spray drying exerts shear forces on nanoparticles and could disassemble polyplexes. Hence, DLS measurements were performed before and after spray drying to visualize any possible effects. Therefore, microparticles were dissolved in HPW for nanoparticle redispersion to mimic impaction and dissolution in the lung. As demonstrated in Figure 2, Z-average values of freshly prepared and redispersed polyplexes do not differ from each other statistically. Also, differences in PDI were not observed. However, we recognized high PDI values which might be explained to some extent by sugar/sugar alcohol monomers. It was shown by Weinbuch et al. that monomers of sugar and sugar alcohol are visible in highly concentrated solutions. [31] And indeed, we accordingly detected monomers at around 1 nm which are not visible if polyplexes are prepared solemnly in HPW (SI-Figure1). This hypothesis is underlined by the fact that with increasing amount of excipient the peak at 1 nm increases (SI-Figure1). This phenomenon also explains the trend of higher PDI values at higher concentrated excipient solutions. Nonetheless, Z-averages and PDI did not differ significantly within one excipient group. However, trehalose formulated polyplexes showed higher PDI values than mannitol formulated polyplexes which might be again attributed to a higher monomer content. In summary, polyplex size and distribution were not affected by spray drying within each formulation. Furthermore, to confirm integrity and hence the base pair length of siRNA in all formulations, a gel assay was executed. Figure 3 shows that independent of the chosen formulation and T-In and T-Out respectively, siRNA was intact in all cases reflected by all bands being detected at the same base pair length. Bands with a smaller molecular weight, which would indicate degradation of siRNA, were not detected. Smears accompanying all spray dried samples are attributed to heparin as shown by similar effects in the sample containing free siRNA mixed with heparin but not in the sample containing free siRNA without heparin. Hence, we state that the base pair length of siRNA was not influenced by spray drying.

However, Z-average, PDI and base pair length are considered qualitative approaches to determine successful spray drying whereas determination of siRNA and polymer content are quantitative and hence extremely relevant for correct dosing.

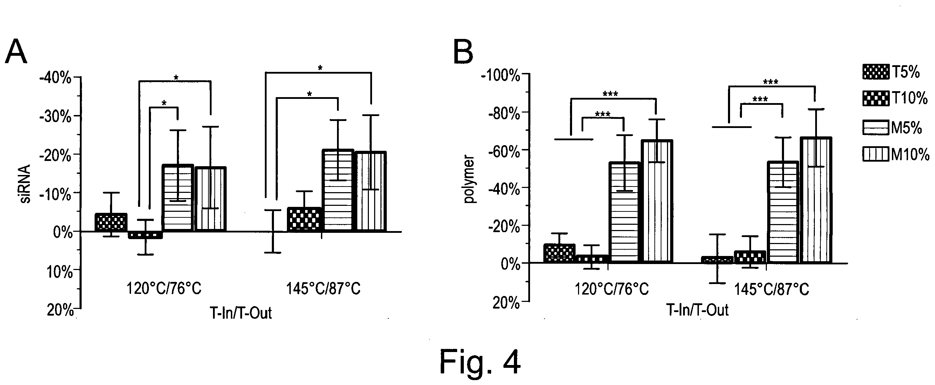

Therefore, the content of siRNA was analyzed in redispersed particles and considerable losses were found when polyplexes were spray dried in the presence of mannitol (Figure 4A). Although not significant but worth mentioning is the fact that losses increased from approximately 17% to 21% when the temperature was increased. This is reasonable as a higher product temperature could lead to greater losses as explained earlier. These findings are also in line with a publication from Wu et al. where naked siRNA was spray dried in presence of mannitol at T-Out between 60 and 125°C. [22] However, when polyplexes were spray dried with trehalose there was no significant change detectable compared to the applied amount of siRNA. Hence, statistically significant differences in regard to siRNA recovery after spray drying between the two different matrix formulations at both spray drying temperatures were observed. As analyzed by two way ANOVA, the choice of excipient was identified as the source of variation accounting for 58.6% of total variation and a p-value of 0.0013 indicating a highly significant effect. Similarly, polymer quantification resulted in no difference in recovery after spray drying when polyplexes were formulated with trehalose but showed losses of approximately 53% and 65% when formulated with 5% mannitol or 10% mannitol, respectively. Here, no statistical differences between both temperatures and concentrations could be detected within each excipient group. However, all mannitol formulations showed significantly higher losses in polymer than their trehalose formulated counterparts. Hence, mannitol formulations were outperformed by their trehalose formulated counterpart in respect to siRNA and polymer recoveries.

This observation might be explained by the fact that trehalose formulations tend to form amorphous particles whereas mannitol tends to crystallize upon spray drying. It was shown by several other publications that amorphous structures can stabilize bio macromolecules during drying. [32-35] Although this effect was not shown before in literature for polyplexes, it is not surprising that trehalose stabilizes polyplexes, also. One explanation for this phenomenon is the water replacement theory. [36] During desiccation trehalose stabilizes the structure of the entrapped molecules by forming hydrogen bonds and maintaining the three-dimensional structure. Also, higher residual moisture content of trehalose formulations might add to this fact enabling greater stabilization by forming additional hydrogen bonds and acting as a plasticizer. In contrast, crystalline mannitol was shown to inefficiently stabilize biopharmaceuticals and might not protect the formulation from a drying-stress induced strand dissociation or potential degradation of the double stranded siRNA. [37] The dissociation of double stranded siRNA due to temperatures close to the melting point could have led to the decreased detection of siRNA with the intercalation based fluorescence quantification as described in chapter 2.12 which does not detect single stranded short RNA. This proposed mechanism is reinforced by the fact that detection of smaller double stranded nucleic acid strains, which would be found if double strand breaks had occurred, could not be detected (Figure 3).

Microparticle Characteristics

Pulmonary delivery via dry powder formulation requires low residual moisture in order to avoid aggregation processes. Although it was discussed above that residual moisture may act as a plasticizer stabilizing polyplexes during the spray drying process, it could nonetheless cause microparticle aggregation and could be a source of microbiological instability and RNase contamination. Therefore, the water content of all formulations was measured by Karl Fischer titration. As reflected in Table 1, trehalose formulations exhibited between 4.6 and 3.8% whereas mannitol formulations showed between 0.4 and 0.2% residual moisture. These results were expected and are in line with results from literature. [38-40]

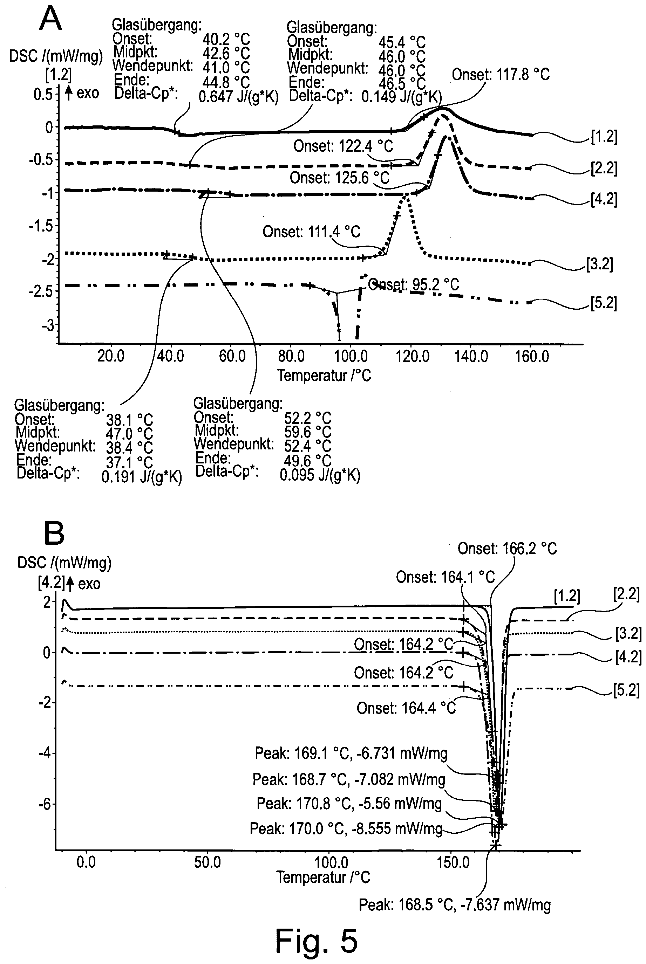

Trehalose commonly solidifies upon spray drying in an amorphous state which was confirmed via DSC (Figure 5). In addition with the hygroscopic nature of trehalose, the reason for the formation of amorphous structures is the fast drying step which does not provide sufficient time for trehalose molecules to arrange within an ordered structure with subsequent crystal nucleation and growth. [41] All of the trehalose formulations showed glass transitions at temperatures between 38° and 53°C corresponding to their residual moisture content (Table 1).

| | Residual moisture (%) |
|---|---|
| T5-120° | 4.53 ± 0.20 |
| T5-145° | 3.85 ± 0.05 |
| T10-120° | 4.61 ± 0.17 |
| T10-145° | 3.82 ± 0.04 |
| M5-120° | 0.40 ± 0.03 |
| M5-145° | 0.27 ± 0.04 |
| M10-120° | 0.26 ± 0.02 |
| M10-145° | 0.19 ± 0.03 |

Table 1

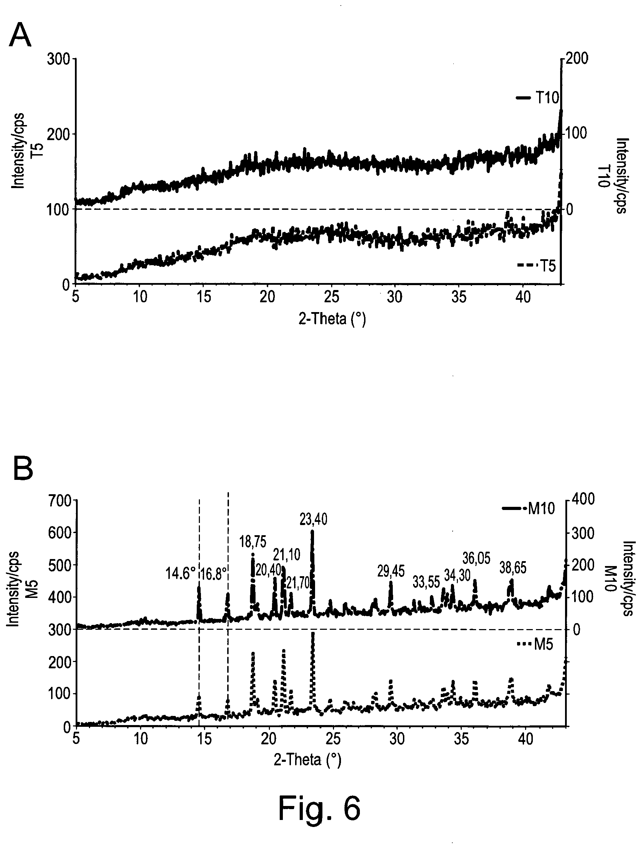

This temperature (Tg) is important for stability predictions during storage as amorphous solid forms are thermodynamically unstable and tend to crystallize if stored close to or above Tg. [42] As discussed above regarding siRNA recovery after spray drying, the amorphous state of the formulation is favorable for polyplex preservation. Hence, for storing these products at room temperature or in the fridge for a longer period of time, high Tg values are necessary. This however is closely linked to the water content: the higher the residual moisture the lower Tg. [43] Also, with a lower residual moisture content degradation processes are less likely to occur. [43] It is therefore of great interest to further decrease the amount of residual moisture in trehalose formulations to avoid nucleation and degradation processes over time and in order to maintain the amorphous state of the formulation. While the impact of storage upon polyplex quality (size, PDI) and quantity has not yet been investigated, these aspects are currently under investigation in a greater scheme of optimizing formulation and process parameters. To confirm the amorphous state of trehalose, formulations were also tested by XRPD and typical amorphous halos were detected (Figure 6A). On the other hand, spray dried mannitol formulations exhibited the same temperature profile as the crystalline starting substance with a melting peak at 170°C as investigated by DSC (Figure 5B). This finding indicates a crystalline form of mannitol. For distinct differentiation between the mannitol polymorphs which could possibly appear, XRPD was performed on formulations prepared at T-In of 145°C. For both tested mannitol formulations, peaks at 14.6° and 16.8° 2-theta were detected which are both linked to the β form of mannitol (Figure 6B). [41, 44] Peaks specific for the α or δ form were not detected indicating that after spray drying β mannitol is the predominant polymorph. Although the drying time of spray dried formulations is very short and could have resulted in an amorphous state as obtained with trehalose, mannitol is less hygroscopic and less soluble. These characteristics lead to the fact that solutions of mannitol dry quicker and crystallize during spray drying. As the residual moisture content was lower for formulations prepared at higher temperatures and no significant differences in nanoparticle characteristics were found between formulations prepared at the two different temperatures, all following experiments were conducted with formulations produced at a T-In of 145°C.

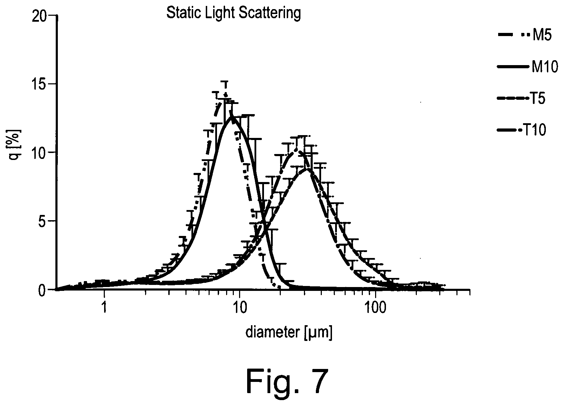

To get a first understanding of microparticle size characteristics, static light scattering was performed in diethyl ether in which neither mannitol nor trehalose is soluble. As visualized in Figure 7 formulations differ from each other depending on the nature of excipient they are made of: both mannitol formulations show significantly lower values in the 10, 50 and 90 percentiles compared to the trehalose formulations (data not shown). Mannitol formulations exhibited geometric median sizes of around 7 and 8 $\mu$m for 5 and 10% formulations, respectively, whereas trehalose formulations showed sizes of around 24 $\mu$m (Table 2).

| | Geometric median diameter (µm) | MMAD (µm) | GSD (µm) | FPF (%) | FPD (µg) |
|---|---|---|---|---|---|
| T5-145° | 24.13 ± 1.47 | 4.65 ± 0.14 | 1.88 ± 0.07 | 14.0 ± 2.4 | 0.15 ± 0.09 |
| T10-145° | 24.02 ± 1.85 | 5.19 ± 0.47 | 1.95 ± 0.04 | 18.3 ± 5.0 | 0.11 ± 0.03 |
| M5-145° | 6.77 ± 0.35 | 4.77 ± 0.15 | 1.94 ± 0.03 | 32.3 ± 5.3 | 0.54 ± 0.05 |
| M10-145° | 8.02 ± 1.11 | 5.50 ± 0.29 | 1.97 ± 0.06 | 22.5 ± 2.4 | 0.21 ± 0.07 |

Table 2

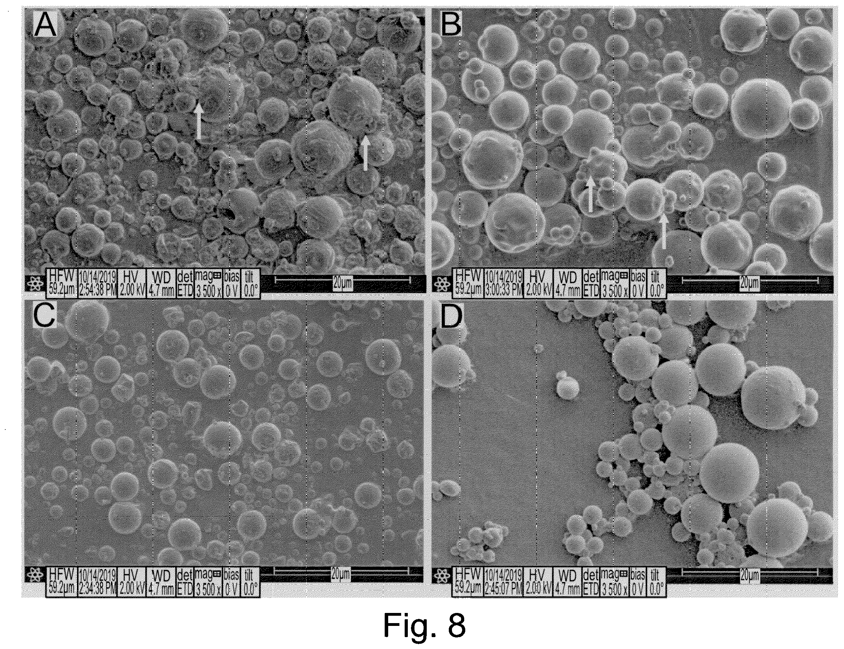

This can be explained by the fact, that amorphous trehalose particles with higher residual moisture show a tendency towards particle fusion through water bridges. [45] Hence, although in fact particles were produced with similar diameters as in the mannitol formulations, as confirmed by SEM (Figure 8), these particles aggregate and may form greater secondary particles. This aggregation can be appreciated in the SEM micrographs (Figure 8). Whereas recordings of mannitol particles confirm the findings of SLS measurements, trehalose particles show much smaller diameters. Additionally, water bridges can be observed as indicated by white arrows. Again, reductions in the water content of trehalose microparticles could considerably reduce this effect. Concerning the surface of the particles, mannitol formulations in general exhibited a very smooth round structure. Trehalose particles also showed nice smooth surfaces when formulated at 10% whereas 5% formulations indicated a somewhat roughened surface.

For pulmonary delivery aerodynamic sizes between 1 and 5 $\mu$m are crucial for successful delivery as discussed above. Although geometric median sizes were greater than 5 $\mu$m, the aerodynamic diameter also depends on the particle density and porosity and might therefore be lower as the geometric diameter. [46] In fact, measurements of impacted siRNA in the NGI revealed MMAD values close to or below 5 $\mu$m for trehalose and mannitol formulations indicating successful lung delivery. Also, calculation of GSD suggests a particle distribution which can be considered for pulmonary application. The FPF is considered as the percentage of drug that was delivered in particles below 5 $\mu$m compared to the overall impacted drug on all stages of the NGI. The higher the FPF the more drug could potentially be delivered to the site of action. Accordingly, fine particle fractions with approximately 27% for both trehalose and approximately 37% for both mannitol formulations, respectively, are considered very good. While only small amounts of particles collected in the NGI were below 5 $\mu$m and could potentially reach bronchioles and alveoli, asthma is a bronchial disease, and deposition in the larger airways may be achieved with particles larger than 5 $\mu$m also. However, to optimize the deposited dose and decrease side potential side effects, decreases in MMAD would be favorable. Compared to the FPF, the FPD is considered as the dose of drug in $\mu$g which was actually delivered in particles with less than 5 $\mu$m. It is therefore not a ratio but an exact dose which could be applied. Here, FPD differed between mannitol and trehalose formulations: whereas mannitol formulations could potentially deliver more than 0.5 $\mu$g, only less than 0.2 $\mu$g of siRNA could currently be delivered by trehalose formulations described above. Although the FPF in trehalose formulated polyplexes were much smaller, this finding cannot solely explain the discrepancy in FPD. To explain this phenomenon, it has to be taken into account that for FPF calculations only the deposited amount of drugs inside the NGI is considered. Depositions in the pre-separator, induction port or even in the Handihaler device itself are not considered. And indeed, a large amount of powder was found on the inner walls of the application device's capsule rack which unfortunately could not be quantified. This finding was noticed only for trehalose and not for mannitol formulations. The most reasonable explanation for this observation might be the amorphous structure of the powder and the higher residual moisture of trehalose formulations. After the release of powder from the capsule through the vacuum and the thus generated centrifugal forces, particles are forced to leave the Handihaler by following the airstream. Smaller and hence lighter particles can follow the airstream directly, whereas a great portion of particles which might be aggregated will follow the airstream only after impacting on and bouncing off the capsule rack's inner wall, thereby possibly disaggregating. However, due to the high residual water content and the subsequent plasticity of trehalose particles, a large amount of powder could adhere to the wall remaining within the device. This explains why suitable values were calculated for MMAD and GSD and FPF of -27% based on the amount of drug deposited inside the NGI from the trehalose formulations. Mannitol powders, on the other hand, were not detected in the capsule rack and hence are suggested to be successfully introduced into the NGI without further losses.

From the overall assessment of the microparticle properties, we therefore conclude that crystalline structures outperform amorphous substances in all analyzed properties even if the amorphous state seemed favorable with regard to polyplex stability.

In Vitro Performance

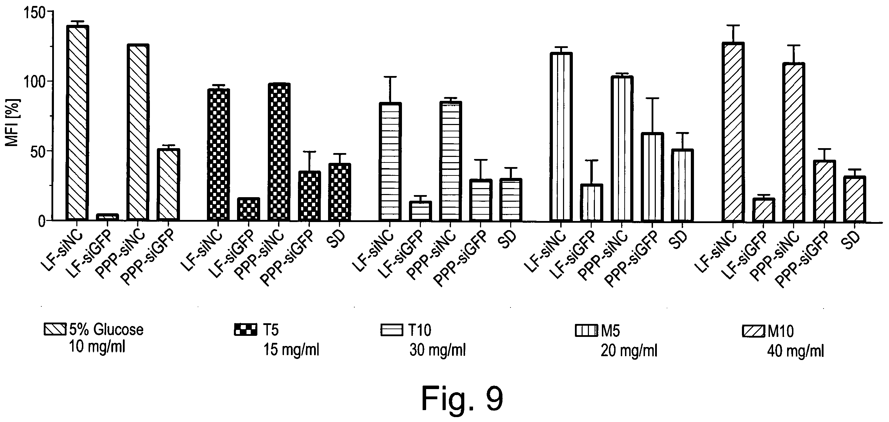

For siRNA delivery it is fundamental to maintain the molecule's bioactivity. Hence, spray dried powder was reconstituted, and enhanced green fluorescence protein expressing H1299 cells were transfected with redispersed polyplexes. Lipofectamine (LF) is a standard transfecting agent *in vitro* and is used as a positive control. [47] It is used to show the maximal possible effect of nucleic acids i.e. here siRNA downregulation of mRNA and subsequent protein expression (GFP). In all cases siRNA was active as the expression of GFP was significantly decreased (Figure 9). Interestingly, lipofectamine-siGFP complexes used in the standard in vitro formulation with 5% glucose showed the highest downregulation observed in all five groups which is >95% in relative reduction (compared to LF-siNC containing a negative control siRNA sequence). Lipofectamine formulated with trehalose or mannitol with the respective concentration of excipient although successful in downregulation, only showed a relative reduction between 70 and 80 %. One possible mechanism for this discrepancy might be explained by the fact, that transfection is a process which is energy consuming and factors which provide energy to cells such as glucose are therefore favorable. [48] Exchanging glucose for trehalose or mannitol might therefore lower this positive effect and cells might take up particles less efficiently than in the glucose reference group. Whether this effect or the viscosity of the matrix solutions used here causes the decreased transfection remains unclear and is part of future research.

In previous studies polyplexes made of PPP and siRNA already showed knockdown efficiencies of -50% *in vitro* [49] and even > 70% *in vivo* where Lipofectamine is not stable and too toxic. [9] Hence, it is of great importance to retain transfection ability and efficiency during spray drying for *in vitro* experiments and follow up studies *in vivo*. As demonstrated in Figure 9, polyplexes formed of siGFP and PPP performed better than their negative control formulations (PPP-siNC). Importantly, all spray dried polyplexes (SD) performed as well as their freshly prepared counterparts. This effect is independent of the excipient's nature and its concentration (marked in grey).

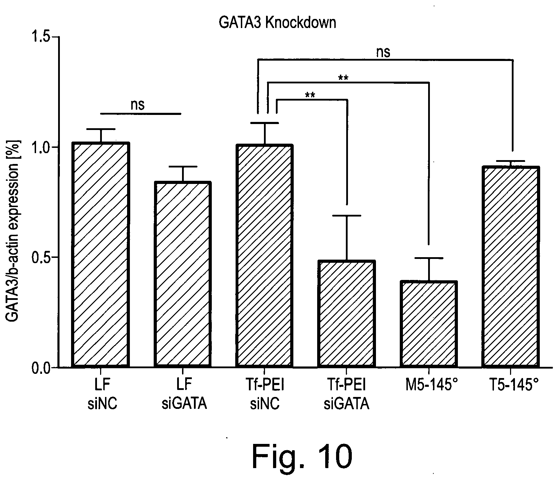

After confirming bioactivity, this knowledge was transferred towards a clinically more relevant model in which GATA binding protein 3 (GATA3) was attempted to be downregulated in CD4+ T cells. In severe uncontrolled asthma, Th2 cells play a crucial role in the activation of downstream effects which orchestrate the full manifestation of asthma which is caused by a upregulated expression of GATA3. [50] Downregulation of such overexpressed proteins could lead to a significant improvement and thus benefit in therapy and administration frequency as shown for other siRNA based therapies. [51] Therefore, the delivery system our group has optimized for T cell transfection of siRNA, namely transferrin-coupled PEI (Tf-PEI), [52] was spray dried in combination with two siRNA sequences targeting GATA3. The amount of siGATA was quantified as described earlier to ensure the amount for transfection. After two days of incubation, the amount of GATA3 mRNA was analyzed and normalized to $\beta$-actin. As expected, Lipofectamine showed no significant difference between the negative control siRNA and siGATA (Figure 10). Freshly prepared polyplexes consisting of Tf-PEI and siGATA however, mediated a significant reduction in the expression of GATA3, and most of all polyplexes of Tf-PEI and siGATA which were spray dried with 5% mannitol (M5-145°) showed identical effects. Importantly, the gene silencing efficacy of M5-145° did not differ significantly from that of freshly prepared siGATA containing polyplexes confirming retained bioactivity and transfection efficiency. Polyplexes spray dried with 5% trehalose however, showed no transfection at all. This is surprising as it was expected that amorphous substances such as trehalose would stabilize transferrin, a 79 kDa protein, to a greater extent than the crystalline mannitol especially because the latter excipient is known to induce protein aggregation upon spray drying. [53] However, here, mannitol formulations stabilized transferrin just as well and allowed successful transfection. One plausible reason for the lack of gene silencing of the trehalose formulation might be redispersion problems in rather small volumes used for transfection considering their aggregation tendency shown in Figure 8. Successful stabilization of transferrin in the mannitol formulations could potentially be achieved by PEI in the polyplexes. PEI participates in the formation of polyplexes but could also interact within transferrin directly. And indeed, as was shown, PEI is able to physically crosslink a protein and increase its stability during stresses induced by pH shifts and stirring. [54] This could explain the maintained stability and conformation of Tf enabling recognition of Tf-receptors of T cells with the Tf decorated polyplexes. Further research will focus on the formation of protein aggregates, the secondary structure and binding kinetics of Tf when spray dried alone, in mixture with PEI and as a Tf-PEI conjugate in either mannitol or trehalose for a better understanding of the processes underlying these findings.

Additional Spray Drying Step

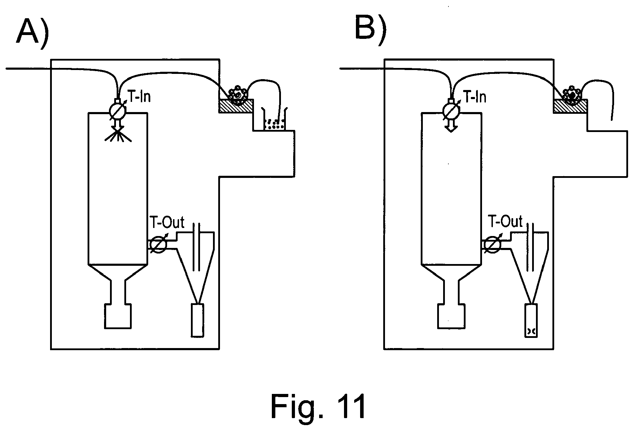

For one spray drying run, a total amount of 30.00 $\mu$g siGFP is diluted with a 5% trehalose solution in RNAse free water to a total volume of 250 $\mu$l. Polyplexes are prepared at N/P ratio of 10. Therefore, the required amount of PPP [1] is also diluted with a 5% trehalose solution in RNase free water to a volume of 250 $\mu$l. The PPP solution is added to the siGFP solution and pipetted vigorously up and down. It is incubated for 20 minutes for polyplexes formation. Subsequently, 4,500 $\mu$l of 5% trehalose solution are added to the polyplex solution. The tube is carefully turned to mix. In the meantime, the spray dryer is turned on; the parameters are set at 145°C inlet temperature, 70% aspirator, an airflow of 40 mm (473 NL/h) and water cooling of the nozzle. RNase free water is spray dried at 1.4 mL/min. If a constant inlet temperature of 145°C is reached, the spray drying process with the polyplex-trehalose dispersion begins. At the end of the process, the sample tube is replaced with RNase free water which is spray dried for another 2 minutes. Hereinafter, the pump is stopped. The spray drying process is finished, and the subsequent drying of the nano-embedded microparticles starts. A timer is set to five minutes and all other parameters are kept constant. Therefore, the subsequent drying takes place at 145°C inlet temperature, as indicated in Figure 11. Finally, the spray drying process is stopped, and the spray dried powder collected.

After the spry drying process, all powder is collected, weighed, and aliquots are used for further analysis. To quantify the PPP and siGFP content within the microparticle powder following spray drying, an aliquot of the powder was dissolved in HPW (Highly purified water), and heparin competition assays were performed to disassociate the siGFP-PPP polyplexes within the NIM powder. Subsequently, the siGFP that was released from the polyplexes was determined by a modified SYBR® gold assay [1] while the PPP concentration was determined by TNBS assay [2]. First, the batch of NIM powder was dissolved in 2 mL HPW in a volumetric flask. Following procedure was executed in triplicates: According to the theoretical content of siGFP, a volume was chosen to achieve approximately 0.04 $\mu$g of siGFP per sample. This solution was diluted to a final volume of 150 $\mu$L per sample with HPW in a tube. Afterwards, 75 $\mu$L of a 58 k IU heparin solution was added to each tube to achieve complete disassociation of polyplexes after 2 h. This solution was further diluted to 450 $\mu$L and distributed into a white 96 well plate (Thermo Scientific™ BioLite microwell plate, Thermo Scientific GmbH, Schwerte, Germany) in triplicates of 100 $\mu$L each. For quantification of siGFP, 30 $\mu$L of a 4 × SYBR® gold solution was added to each well and incubated for 10 min in the dark at room temperature. The fluorescence of each sample was quantified using a Synergy 2 multi-mode microplate reader (BioTek Instrument, Winooski, VT, U.S.A.) at excitation wavelength of 485/20 nm and emission wavelength of 520/20 nm and compared to a freshly prepared standard curve of free siGFP incubated with SYBR® gold dye. For quantification of PPP, 100 $\mu$L of batch dissolved solution in 2 mL borax buffer 0.1 M (Sodium Borax Decahydrate, Sigma Aldrich, Germany) was incubated with 30 $\mu$L

of 3 mM TNBS solution in triplicates and absorbance at 405 nm was determined after a 1 h incubation using a Synergy 2 multi-mode microplate reader (BioTek Instrument, Winooski, VT, U.S.A.). The measured absorbance was compared to a freshly prepared standard curve of free PPP incubated with TNBS reagent. To verify each quantification assay, internal standards were prepared freshly and analyzed in parallel. These standards consisted of siGFP and PPP of known amounts which were chosen according to the theoretical amount of siGFP or PPP being analyzed in the sample, respectively. These were 0.04 µg siGFP per well for nucleic acid quantification and 0.48 to 1.2 µg PPP for polymer quantification. Measurements with deviations of less than 10% of the internal standard were considered as precise and taken into account for further analysis.

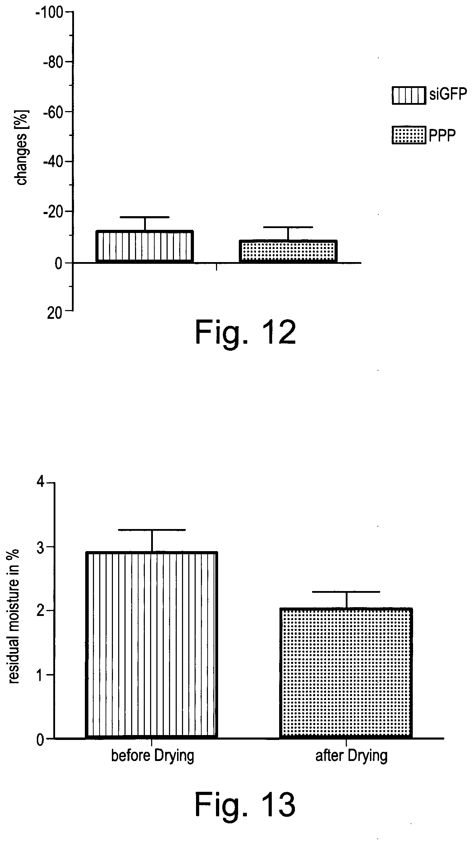

The analysis of spray dried powders after additional drying revealed that during the spray drying process, about 10% of siRNA and polymer were lost as shown in Figure 12. The corresponding losses support stability of the polyplexes during the spray drying process and confirm that no additional material is lost during the additional drying step.

The residual water content of the NIM powder after spray drying was determined by coulometric measurement using an Aqua 40.00 Karl Fischer Titrator with corresponding software from Analytik Jena AG (Jena, Germany) as shown in Figure 13. The samples (approx. 15 mg) were loaded into 2R vials, placed into the heating chamber and measured at 100 °C until the measurement drift reached the start drift $\leq$+2 µg/min or until a final measurement time of 10 min. The start drift was established after approximately 1 h of equilibration showing a rate of less than 10 µg/min. Hydranal Coulomat AG (Riedel-deHaën, Seelze, Germany) was used as reagent. Before each session, the titrator was calibrated with a 1% water standard. The moisture content was calculated as the % weight of water relative to the overall sample weight.

The analysis of the powders before and after the additional drying step confirmed that a statistically significant reduction in residual moisture was achieved by the additional drying step. Accordingly, also the fine particle fraction (FPF) of the trehalose formulations was increased after the additional drying step. Similar results are likely for other amorphous excipients.

This again increases the fine particle fraction of the powder, i.e. the fraction with aerodynamic diameters (MMAD) of less than 5µm. Based on this improved small particle fraction, the pharmaceutically active dose of the powder can be greatly improved.

Summary

In summary, according to the invention spray drying above the melting temperature of naked siRNA results in significant changes in the quantity of siRNA recovered after spray drying, even when the siRNA is present in polyplexes encapsulated in matrices of excipients. Inventive spray drying at or below the melting temperature show no significant differences in respect to recovered quantity and base pair length. Furthermore, it was surprisingly found that the tubing material can have a tremendous effect on the preparation and processing of spray dried polyplexes as interactions between the tubing material and siRNA seem to occur on a large scale.

We demonstrated that spray drying did not affect polyplex size and PDI independent of the different excipients and their concentration used in this study. This was also shown for siRNA integrity. Quantitative analysis revealed significant losses of siGFP and PPP after spray drying when formulated with mannitol, a representative for crystalline substances. However, no changes regarding the recovery of both polyplex components were observed when spray dried with trehalose, the typical matrix used for amorphous microparticles. Therefore, it is believed that for nanoparticle properties amorphous substances are crucial to minimize losses through processing.

An additional drying step exhibits positive effects on the spray dried powder without causing a significant increase in siRNA losses. The resulting powder shows a decrease in residual moisture content and exhibits improved microparticle characteristics without lowering nanoparticle stability.

Concerning microparticle characteristics, mannitol formulations significantly outperformed trehalose formulations with regard to particle characteristics. Due to crystalline structure and subsequent lower residual moisture, mannitol particles exhibited smaller geometric median sizes and showed favorable aerodynamic characteristics.

For in vitro analysis, both formulations showed efficient downregulation of GFP in an eGFP expressing cell line indicating preserved bioactivity with all tested formulations. These findings were translated towards primary CD4+ T cells which play a central role in the pathogenesis of inflammatory diseases such as asthma where GATA3 upregulation can be observed. We demonstrated that spray drying of polyplexes had no negative effect on the efficiency when formulated with mannitol, and successful transfection of primary T cells *ex vivo* was achieved with the spray dried mannitol formulation for dry powder inhalation as reflected by efficient and sequence specific GATA3 silencing.

It is an advantageous embodiment of the current invention to combine beneficial properties of both crystalline and amorphous particles for stabilization through the amorphous matrix and optimal microparticle characteristics through the crystalline material.

References

[1] S. Weber, A. Zimmer, J. Pardeike, Solid Lipid Nanoparticles (SLN) and Nanostructured Lipid Carriers (NLC) for pulmonary application: a review of the state of the art, Eur. J. Pharm. Biopharm., 86 (2014) 7-22.

[2] A. Ray, A. Mandal, A.K. Mitra, Recent Patents in Pulmonary Delivery of Macromolecules, Recent Pat Drug Deliv Formul, 9 (2015) 225-236.

[3] G.K. Crompton, Dry powder inhalers: advantages and limitations, J. Aerosol Med., 4 (1991) 151-156.

[4] P. Hematti, E.G. Schmuck, J.A. Kink, A.N. Raval, Generation of therapeutic cells using extracellular components of target organs, in, US Patent US20180282698A1 2018.

[5] G.J. Hannon, RNA interference, Nature, 418 (2002) 244-251.

[6] H.J. Kim, A. Kim, K. Miyata, K. Kataoka, Recent progress in development of siRNA delivery vehicles for cancer therapy, Adv. Drug Delivery Rev., 104 (2016) 61-77.

[7] S.S. Titze-de-Almeida, P.R.P. Brandao, I. Faber, R. Titze-de-Almeida, Leading RNA Interference Therapeutics Part 1: Silencing Hereditary Transthyretin Amyloidosis, with a Focus on Patisiran, Mol. Diagn. Ther., (2019) 1-11.

[8] P. de Paula Brandão, S. Titze-de-Almeida, R. Titze-de-Almeida, Leading RNA Interference Therapeutics Part 2: Silencing Delta-Aminolevulinic Acid Synthase 1, with a Focus on Givosiran, Mol. Diagn. Ther., (2019) 1-8.

[9] D.P. Feldmann, Y. Xie, S.K. Jones, D. Yu, A. Moszczynska, O.M. Merkel, The impact of microfluidic mixing of triblock micelleplexes on in vitro in vivo gene silencing and intracellular trafficking, Nanotechnology, 28 (2017) 224001.

[10] S.K. Jones, V. Lizzio, O.M. Merkel, Folate Receptor Targeted Delivery of siRNA and Paclitaxel to Ovarian Cancer Cells via Folate Conjugated Triblock Copolymer to Overcome TLR4 Driven Chemotherapy Resistance, Biomacromolecules, 17 (2016) 76-87.

[11] T. Endres, M. Zheng, M. Beck-Broichsitter, T. Kissel, Lyophilised ready-to-use formulations of PEG-PCL-PEI nano-carriers for siRNA delivery, Int. J. Pharm., 428 (2012) 121-124.

[12] T.W.M. Keil, D.P. Feldmann, G. Costabile, Q. Zhong, S. da Rocha, O.M. Merkel, Characterization of spray dried powders with nucleic acid-containing PEI nanoparticles, Eur J Pharm Biopharm, (2019).

[13] K. Cal, K. Sollohub, Spray drying technique. I: Hardware and process parameters, J. Pharm. Sci., 99 (2010) 575-586.

[14] T. Okuda, M. Morishita, K. Mizutani, A. Shibayama, M. Okazaki, H. Okamoto, Development of spray-freeze-dried siRNA/PEI powder for inhalation with high aerosol performance and strong pulmonary gene silencing activity, J. Control. Release, 279 (2018) 99-113.

[15] M. Cardenas, A. Braem, T. Nylander, B. Lindman, DNA compaction at hydrophobic surfaces induced by a cationic amphiphile, Langmuir, 19 (2003) 7712-7718.

[16] J. Horn, J. Schanda, W. Friess, Impact of fast and conservative freeze-drying on product quality of protein-mannitol-sucrose-glycerol lyophilizates, Eur. J. Pharm. Biopharm., 127 (2018) 342-354.

[17] M.T. Cicerone, M.J. Pikal, K.K. Qian, Stabilization of proteins in solid form, Adv. Drug Delivery Rev., 93 (2015) 14-24.

[18] United-States-Pharmacopeial-Convention, Particle Size - Aerodynamic Size Distribution, in: 601 Aerosols, Nasal Sprays, Metered-Dose Inhalers, and Dry Powder Inhalers, USP 35, 2012, pp. 232-252.

[19] European-Pharmacopoeia-Commission, Aerodynamic Assessment of Fine Particles, in: 2.9.18 Preparations for Inhalation, Ph.Eur. 9.0, 2017, pp. 440-454.

[20] W. Liang, M.Y. Chow, P.N. Lau, Q.T. Zhou, P.C. Kwok, G.P. Leung, A.J. Mason, H.K. Chan, L.L. Poon, J.K. Lam, Inhalable dry powder formulations of siRNA and pH-responsive peptides with antiviral activity against H1N1 influenza virus, Mol. Pharmaceutics, 12 (2015) 910-921.

[21] W.L. Liang, M.Y.T. Chow, S.F. Chow, H.K. Chan, P.C.L. Kwok, J.K.W. Lam, Using two-fluid nozzle for spray freeze drying to produce porous powder formulation of naked siRNA for inhalation, Int. J. Pharm., 552 (2018) 67-75.

[22] J. Wu, L. Wu, F. Wan, J. Rantanen, D. Cun, M. Yang, Effect of thermal and shear stresses in the spray drying process on the stability of siRNA dry powders, Int. J. Pharm., 566 (2019) 32-39.

[23] J. Schulze, S. Kuhn, S. Hendrikx, M. Schulz-Siegmund, T. Polte, A. Aigner, Spray-Dried Nanoparticle-in-Microparticle Delivery Systems (NiMDS) for Gene Delivery, Comprising Polyethylenimine (PEI)-Based Nanoparticles in a Poly(Vinyl Alcohol) Matrix, Small, 14 (2018) e1701810.

[24] L. Liu, M. Zheng, D. Librizzi, T. Renette, O.M. Merkel, T. Kissel, Efficient and Tumor Targeted siRNA Delivery by Polyethylenimine-graft-polycaprolactone-block-poly (ethylene glycol)-folate (PEI-PCL-PEG-Fol), Molecular pharmaceutics, 13 (2015) 134-143.

[24a] Y. Xie, N. Kim, V. Nadithe, D. Schalk, A. Thakur, A. Kilic, D. Bassett, L. Lum, O. Merkel, Targeted delivery of siRNA to activated T cells via transferrin-polythylenimine (Tf-PEI) as a potential therapy of asthma, Journal of Controlled Release, 229 (2016) 120-129

[25] HORIBA, Understanding the Chi Square and R Parameter - Calculations in the LA-950 Software, https://www.horiba.com/fileadmin/uploads/Scientific/Documents/PSA/TN1 53.pdf, 4.

[26] S. Claus, C. Weiler, J. Schiewe, W. Friess, Optimization of the fine particle fraction of a lyophilized lysozyme formulation for dry powder inhalation, Pharm. Res., 30 (2013) 1698-1713.

[27] R. Kandil, Y. Xie, R. Heermann, L. Isert, K. Jung, A. Mehta, O.M. Merkel, Coming in and Finding Out: Blending Receptor-Targeted Delivery and Efficient Endosomal Escape in a Novel Bio-Responsive siRNA Delivery System for Gene Knockdown in Pulmonary T Cells, Advanced therapeutics, 2 (2019) 1900047.

[28] Y.R. Xie, N.H. Kim, V. Nadithe, D. Schalk, A. Thakur, A. Kilic, L.G. Lum, D.J.P. Bassett, O.M. Merkel, Targeted delivery of siRNA to activated T cells via transferrin-polyethylenimine (Tf-PEI) as a potential therapy of asthma, J. Control. Release, 229 (2016) 120-129.

[29] Büchi-Labortechnik-AG, Training Papers Spray Drying, https://static1.buchi.com/sites/default/files/downloads/Set_3_Training_Pap ers_Spray_Drying_en_01.pdf?996b2db24007502bd69c913b675467cfc63 880ba, (1997 - 2002).

[30] M. Terrazas, E.T. Kool, RNA major groove modifications improve siRNA stability and biological activity, Nucleic Acids Res., 37 (2009) 346-353.

[31] D. Weinbuch, J.K. Cheung, J. Ketelaars, V. Filipe, A. Hawe, J. den Engelsman, W. Jiskoot, Nanoparticulate Impurities in Pharmaceutical-Grade Sugars and their Interference with Light Scattering-Based Analysis of Protein Formulations, Pharm. Res., 32 (2015) 2419-2427.

[32] N.K. Jain, I. Roy, Effect of trehalose on protein structure, Protein Sci., 18 (2009) 24-36.

[33] I. Vollrath, W. Friess, A. Freitag, A. Hawe, G. Winter, Comparison of ice fog methods and monitoring of controlled nucleation success after freeze-drying, Int. J. Pharm., 558 (2019) 18-28.

[34] J.H. Gitter, R. Geidobler, I. Presser, G. Winter, A comparison of controlled ice nucleation techniques for freeze-drying of a therapeutic antibody, J. Pharm. Sci., 107 (2018) 2748-2754.

[35] J.H. Crowe, L.M. Crowe, D. Chapman, Preservation of membranes in anhydrobiotic organisms: the role of trehalose, Science, 223 (1984) 701-703.

[36] J.H. Crowe, F.A. Hoekstra, L.M. Crowe, Anhydrobiosis, Annu. Rev. Physiol., 54 (1992) 579-599.

[37] K. Izutsu, S. Kojima, Excipient crystallinity and its protein-structure-stabilizing effect during freeze-drying, Journal of pharmacy and pharmacology, 54 (2002) 1033-1039.

[38] M. Adler, G. Lee, Stability and surface activity of lactate dehydrogenase in spray-dried trehalose, J. Pharm. Sci., 88 (1999) 199-208.

[39] M. Maury, K. Murphy, S. Kumar, L. Shi, G. Lee, Effects of process variables on the powder yield of spray-dried trehalose on a laboratory spray-dryer, Eur. J. Pharm. Biopharm., 59 (2005) 565-573.

[40] N.Y. Chew, H.-K. Chan, Influence of particle size, air flow, and inhaler device on the dispersion of mannitol powders as aerosols, Pharm. Res., 16 (1999) 1098-1103.

[41] Y.Y. Lee, J.X. Wu, M. Yang, P.M. Young, F. van den Berg, J. Rantanen, Particle size dependence of polymorphism in spray-dried mannitol, Eur. J. Pharm. Sci., 44 (2011) 41-48.

[42] K.J. Geh, M. Hubert, G. Winter, Progress in formulation development and sterilisation of freeze-dried oligodeoxynucleotide-loaded gelatine nanoparticles, Eur. J. Pharm. Biopharm., 129 (2018) 10-20.

[43] E.Y. Shalaev, G. Zografi, How does residual water affect the solid-state degradation of drugs in the amorphous state?, J. Pharm. Sci., 85 (1996) 1137-1141.

[44] J.H. Gitter, R. Geidobler, I. Presser, G. Winter, Significant Drying Time Reduction Using Microwave- Assisted Freeze- Drying for a Monoclonal Antibody, J. Pharm. Sci., 107 (2018) 2538-2543.

[45] L. Weng, S. Ziaei, G.D. Elliott, Effects of Water on Structure and Dynamics of Trehalose Glasses at Low Water Contents and its Relationship to Preservation Outcomes, Sci. Rep., 6 (2016) 28795.

[46] I. Gonda, A.F. Abdelkhalik, On the Calculation of Aerodynamic Diameters of Fibers, Aerosol Sci. Technol., 4 (1985) 233-238.

[47] F. Cardarelli, L. Digiacomo, C. Marchini, A. Amici, F. Salomone, G. Fiume, A. Rossetta, E. Gratton, D. Pozzi, G. Caracciolo, The intracellular trafficking mechanism of Lipofectamine-based transfection reagents and its implication for gene delivery, Sci. Rep., 6 (2016) 25879.

[48] J.S. Kim, T.J. Yoon, K.N. Yu, M.S. Noh, M. Woo, B.G. Kim, K.H. Lee, B.H. Sohn, S.B. Park, J.K. Lee, Cellular uptake of magnetic nanoparticle is mediated through energy-dependent endocytosis in A549 cells, J. Vet. Sci., 7 (2006) 321-326.

[49] Y. Liu, O. Samsonova, B. Sproat, O. Merkel, T. Kissel, Biophysical characterization of hyper-branched polyethylenimine-graft-polycaprolactone-block-mono-methoxyl-poly (ethylene glycol) copolymers (hy-PEI-PCL-mPEG) for siRNA delivery, J. Control. Release, 153 (2011) 262-268.

[50] A. Ray, L. Cohn, Th2 cells and GATA-3 in asthma: new insights into the regulation of airway inflammation, J. Clin. Invest., 104 (1999) 985-993.

[51] S.M. Hoy, Patisiran: First Global Approval, Drugs, 78 (2018) 1625-1631.

[52] R. Kandil, Y. Xie, R. Heermann, L. Isert, K. Jung, A. Mehta, O.M. Merkel, Coming in and Finding Out: Blending Receptor-Targeted Delivery and Efficient Endosomal Escape in a Novel Bio-Responsive siRNA Delivery System for Gene Knockdown in Pulmonary T Cells, Adv. Ther. (Weinheim, Ger.), 2 (2019) 1900047.

[53] S. Schüle, W. Frieß, K. Bechtold-Peters, P. Garidel, Conformational analysis of protein secondary structure during spray-drying of antibody/mannitol formulations, Eur. J. Pharm. Biopharm., 65 (2007) 1-9.

[54] C. Garcia-Galan, O. Barbosa, R. Fernandez-Lafuente, Stabilization of the hexameric glutamate dehydrogenase from Escherichia coli by cations and polyethyleneimine, Enzyme Microb Technol, 52 (2013) 211-217.

[55] J. Horn, E. Tolardo, D. Fissore, W. Friess, Crystallizing amino acids as bulking agents in freeze-drying, Eur. J. Pharm. Biopharm., 132 (2018) 70-82.

[56] R.E. Johnson, C.F. Kirchhoff, H.T. Gaud, Mannitol-sucrose mixtures-versatile formulations for protein lyophilization, J. Pharm. Sci., 91 (2002) 914-922.

[57] A. Hawe, W. Friess, Physicochemical characterization of the freezing behavior of mannitol-human serum albumin formulations, AAPS PharmSciTech, 7 (2006) 94.

## Claims

1. Method for producing high yield nano-in-micro (NIM) encapsulated bioactive siRNA dry powder comprising the steps of:

   - providing an aqueous suspension comprising polyplexes, in particular polyelectrolyte complexes, formed from at least a polyamine, and/or polyamide and/or polyester and siRNA, wherein the polyplexes are provided with and/or encapsulated into water soluble excipients, in particular highly purified water and/or sugar alcohol and/or sugar;
   - spray drying the aqueous suspension using a spray drying apparatus, preferably a Büchi B-290, by feeding the aqueous suspension to a spray drying atomizing nozzle and subjecting the atomized droplets to a heated gas stream of a carrier gas, preferably dried and cleaned air, in particular through a multicomponent atomizing nozzle for the suspension and an atomizing gas;
   - collecting a spray dried powder in an accumulation means of the spray drying apparatus

   **characterized in that**
   the temperature in the vicinity of an outlet opening of the spray drying apparatus, in particular an outlet opening connecting a spray drying chamber with the accumulation means, during feeding of the aqueous suspension to the nozzle is controlled by temperature controlling means to be limited to an upper threshold temperature which is equal to or below a melting temperature of the respective naked siRNA.

2. Method according to claim 1,
   **characterized in that**,
   the upper threshold temperature is set to 90°C, in particular 80°C.

3. Method according to claim 1 or 2,
   **characterized in that**,
   the aqueous suspension is fed to the nozzle through a high grade tubing material, in particular a high grade silicon based tubing material, especially Pumpsil ®.

4. Method according to claims 1 to 3,
   **characterized by**
   an additional drying step, in which a carrier gas is fed through the spray drying apparatus, especially the atomizing nozzle, without being mixed with aqueous suspension and in particular while the spray dried powder has already been transferred to the accumulation means.

5. Method according to claim 4,
   **characterized in that**,
   the additional drying step is carried out with the temperature in the vincinity of the spry dry nozzle being controlled by temperature controlling means to be limited to an upper threshold temperature, in particular an upper threshold temperature of 90°C, in particular 80°C.

6. Method according to any of the claims 1 to 5,
   **characterized in that**,
   the additional drying step is performed for 3 to 10 minutes, in particular 4 to 6 minutes, especially for 1 to 1.5 minutes per 10μg siRNA.

7. Method according to claims 5 or 6,
   **characterized in that**,
   the additional drying step is carried out until a residual moisture of the spray dried powder of less than 3%, in particular less than 2% is reached.

8. Method according to claims 1 to 7,
   **characterized in that** polyethyleneimine-graft-polycaprolactone-block-polyethylene glycol (PEI-g-PCL-b-PEG or PPP) and/or a bioconjugate of polyethylenimine, especially Transferrin conjugated polyethylenimine (Tf-PEI), and/or polyspermine is used as polyamine.

9. Powder containing bioactive siRNA in the form of polyplexes, in particular polyelectrolyte complexes, formed from at least a polyamine, polyamide and/or polyester and siRNA, wherein the polyplexes are encapsulated into water soluble excipients, in particular mannitol and/or trehalose, with a residual moisture of less than 5%, and preferably with aerodynamic diameters (MMAD) between 0,5 and 10μm, preferably 1 and 5μm, produced according to the method of any of claims 1 to 8

10. Powder according to claim 9,
    **characterized in that**,
    the powder can be resuspended in water to polyplexes or polyplex suspensions which are not significantly different, in particular which do not vary more than +/- 10%, from their initial formulation before spray drying in regard to size, polydispersity and zeta potential.

11. Powder according to claim 9 or 10,
    **characterized in that**
    the polyamine is polyethyleneimine-graft-polycaprolactone-block-polyethylene glycol (PEI-g-PCL-b-PEG or PPP) and/or a bioconjuagte of polyethylenimine, in particular Transferrin conjugated polyethylenimine (Tf-PEI), and/or polyspermine.

12. Use of a powder composition or blend comprising at least a powder according to claim 9 to 11 as a pharmaceutical dosage form, especially for pulmonary delivery.

13. Use according to claim 12,
    **characterized in that**,
    the siRNA is active in silencing the translation of messenger RNA into proteins causing lung diseases.

Fig. 1

Fig. 2

Fig. 3

Fig. 4

Fig. 5

Fig. 6

Fig. 7

Fig. 8

Fig. 9

Fig. 10

A)

B)

Fig. 11

Fig. 12

Fig. 13

Europäisches
Patentamt

European
Patent Office

Office européen
des brevets

# EUROPEAN SEARCH REPORT

Application Number

EP 20 20 1629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2018/010815 A1 (BIONTECH RNA PHARMACEUTICALS GMBH [DE] ET AL.) 18 January 2018 (2018-01-18) * example 13 * | 1-13 | INV. A61K9/16 A61K47/69 |
| Y | KEIL T.W.M. ET AL.: "Characterization of spray dried powders with nucleic acid-containing PEI nanoparticles", EUROPEAN JOURNAL OF PHARMACEUTICS AND BIOPHARMACEUTICS, ELSEVIER SCIENCE PUBLISHERS B.V., AMSTERDAM, NL, vol. 143, 21 August 2019 (2019-08-21), pages 61-69, XP085805615, ISSN: 0939-6411, DOI: 10.1016/J.EJPB.2019.08.012 [retrieved on 2019-08-21] * page 62 * | 1-13 | |
| Y | OKUDA T. ET AL.: "Development of Biodegradable Polycation-Based Inhalable Dry Gene Powders by Spray Freeze Drying", PHARMACEUTICS, vol. 7, no. 3, 26 August 2015 (2015-08-26), pages 233-254, XP055541775, DOI: 10.3390/pharmaceutics7030233 * the whole document * | 1-13 | TECHNICAL FIELDS SEARCHED (IPC) A61K |
| Y | WO 2006/008517 A2 (UNIV CARDIFF [GB]; BRICHALL JAMES CARADOC [GB]; LI HAOYING [GB]) 26 January 2006 (2006-01-26) * examples * | 1-13 | |

-/--

The present search report has been drawn up for all claims

| Place of search | Date of completion of the search | Examiner |
|---|---|---|
| Munich | 19 March 2021 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 20 20 1629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MAAS S.G. ET AL.: "The impact of spray drying outlet temperature on the particle morphology of mannitol", POWDER TECHNOLOGY, ELSEVIER, BASEL (CH), vol. 213, no. 1, 25 June 2011 (2011-06-25), pages 27-35, XP028275753, ISSN: 0032-5910, DOI: 10.1016/J.POWTEC.2011.06.024 [retrieved on 2011-07-19] * the whole document * ----- | 1-13 | |

TECHNICAL FIELDS
SEARCHED      (IPC)

The present search report has been drawn up for all claims

| Place of search | Date of completion of the search | Examiner |
|---|---|---|
| Munich | 19 March 2021 | Giménez Miralles, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 20 20 1629

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2021

| Patent document cited in search report | Publication date | Patent family member(s) | | Publication date |
|---|---|---|---|---|
| WO 2018010815 A1 | 18-01-2018 | AU | 2017297822 A1 | 03-01-2019 |
| | | BR | 112018077163 A2 | 30-04-2019 |
| | | CA | 3027148 A1 | 18-01-2018 |
| | | CN | 109562059 A | 02-04-2019 |
| | | EP | 3484445 A1 | 22-05-2019 |
| | | JP | 2019527685 A | 03-10-2019 |
| | | KR | 20190029588 A | 20-03-2019 |
| | | RU | 2019103434 A | 17-08-2020 |
| | | US | 2019321458 A1 | 24-10-2019 |
| | | WO | 2018010815 A1 | 18-01-2018 |
| | | WO | 2018011406 A1 | 18-01-2018 |
| WO 2006008517 A2 | 26-01-2006 | EP | 1778189 A2 | 02-05-2007 |
| | | US | 2008202513 A1 | 28-08-2008 |
| | | WO | 2006008517 A2 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3277743 A4
- WO 2008151150 A2
- US 20020137670 A1
- US 20180282698 A1

### Non-patent literature cited in the description

- **S. WEBER ; A. ZIMMER ; J. PARDEIKE.** Solid Lipid Nanoparticles (SLN) and Nanostructured Lipid Carriers (NLC) for pulmonary application: a review of the state of the art. *Eur. J. Pharm. Biopharm.,* 2014, vol. 86, 7-22
- **A. RAY ; A. MANDAL ; A.K. MITRA.** Recent Patents in Pulmonary Delivery of Macromolecules. *Recent Pat Drug Deliv Formul,* 2015, vol. 9, 225-236
- **G.K. CROMPTON.** Dry powder inhalers: advantages and limitations. *J. Aerosol Med.,* 1991, vol. 4, 151-156
- **G.J. HANNON.** RNA interference. *Nature,* 2002, vol. 418, 244-251
- **H.J. KIM ; A. KIM ; K. MIYATA ; K. KATAOKA.** Recent progress in development of siRNA delivery vehicles for cancer therapy. *Adv. Drug Delivery Rev.,* 2016, vol. 104, 61-77
- **S.S. TITZE-DE-ALMEIDA ; P.R.P. BRANDAO ; I. FABER ; R. TITZE-DE-ALMEIDA.** Leading RNA Interference Therapeutics Part 1: Silencing Hereditary Transthyretin Amyloidosis, with a Focus on Patisiran. *Mol. Diagn. Ther.,* 2019, 1-11
- **P. DE PAULA BRANDÃO ; S. TITZE-DE-ALMEIDA ; R. TITZE-DE-ALMEIDA.** Leading RNA Interference Therapeutics Part 2: Silencing Delta-Aminolevulinic Acid Synthase 1, with a Focus on Givosiran. *Mol. Diagn. Ther,* 2019, 1-8
- **D.P. FELDMANN ; Y. XIE ; S.K. JONES ; D. YU ; A. MOSZCZYNSKA ; O.M. MERKEL.** The impact of microfluidic mixing of triblock micelleplexes on in vitro in vivo gene silencing and intracellular trafficking. *Nanotechnology,* 2017, vol. 28, 224001
- **S.K. JONES ; V. LIZZIO ; O.M. MERKEL.** Folate Receptor Targeted Delivery of siRNA and Paclitaxel to Ovarian Cancer Cells via Folate Conjugated Triblock Copolymer to Overcome TLR4 Driven Chemotherapy Resistance. *Biomacromolecules,* 2016, vol. 17, 76-87
- **T. ENDRES ; M. ZHENG ; M. BECK-BROICHSITTER ; T. KISSEL.** Lyophilised ready-to-use formulations of PEG-PCL-PEI nano-carriers for siRNA delivery. *Int. J. Pharm,* 2012, vol. 428, 121-124
- **T.W.M. KEIL ; D.P. FELDMANN ; G. COSTABILE ; Q. ZHONG ; S. DA ROCHA ; O.M. MERKEL.** Characterization of spray dried powders with nucleic acid-containing PEI nanoparticles. *Eur J Pharm Biopharm,* 2019
- **K. CAL ; K. SOLLOHUB.** Spray drying technique. I: Hardware and process parameters. *J. Pharm. Sci.,* 2010, vol. 99, 575-586
- **T. OKUDA ; M. MORISHITA ; K. MIZUTANI ; A. SHIBAYAMA ; M. OKAZAKI ; H. OKAMOTO.** Development of spray-freeze-dried siRNA/PEI powder for inhalation with high aerosol performance and strong pulmonary gene silencing activity. *J. Control. Release,* 2018, vol. 279, 99-113
- **M. CARDENAS ; A. BRAEM ; T. NYLANDER ; B. LINDMAN.** DNA compaction at hydrophobic surfaces induced by a cationic amphiphile. *Langmuir,* 2003, vol. 19, 7712-7718
- **J. HORN ; J. SCHANDA ; W. FRIESS.** Impact of fast and conservative freeze-drying on product quality of protein-mannitol-sucrose-glycerol lyophilizates. *Eur. J. Pharm. Biopharm.,* 2018, vol. 127, 342-354
- **M.T. CICERONE ; M.J. PIKAL ; K.K. QIAN.** Stabilization of proteins in solid form. *Adv. Drug Delivery Rev,* 2015, vol. 93, 14-24
- United-States-Pharmacopeial-Convention, Particle Size - Aerodynamic Size Distribution, in: 601 Aerosols, Nasal Sprays. *Metered-Dose Inhalers, and Dry Powder Inhalers,* 2012, 232-252
- European-Pharmacopoeia-Commission, Aerodynamic Assessment of Fine Particles, in: 2.9.18 Preparations for Inhalation. *Ph.Eur. 9.0,* 2017, 440-454

- **W. LIANG ; M.Y. CHOW ; P.N. LAU ; Q.T. ZHOU ; P.C. KWOK ; G.P. LEUNG ; A.J. MASON ; H.K. CHAN ; L.L. POON ; J.K. LAM.** Inhalable dry powder formulations of siRNA and pH-responsive peptides with antiviral activity against H1N1 influenza virus. *Mol. Pharmaceutics,* 2015, vol. 12, 910-921
- **W.L. LIANG ; M.Y.T. CHOW ; S.F. CHOW ; H.K. CHAN ; P.C.L. KWOK ; J.K.W. LAM.** Using two-fluid nozzle for spray freeze drying to produce porous powder formulation of naked siRNA for inhalation. *Int. J. Pharm.,* 2018, vol. 552, 67-75
- **J. WU ; L. WU ; F. WAN ; J. RANTANEN ; D. CUN ; M. YANG.** Effect of thermal and shear stresses in the spray drying process on the stability of siRNA dry powders. *Int. J. Pharm.,* 2019, vol. 566, 32-39
- **J. SCHULZE ; S. KUHN ; S. HENDRIKX ; M. SCHULZ-SIEGMUND ; T. POLTE ; A. AIGNER.** Spray-Dried Nanoparticle-in-Microparticle Delivery Systems (NiMDS) for Gene Delivery. *Comprising Polyethylenimine (PEI)-Based Nanoparticles in a Poly(Vinyl Alcohol) Matrix, Small,* 2018, vol. 14, e1701810
- **L. LIU ; M. ZHENG ; D. LIBRIZZI ; T. RENETTE ; O.M. MERKEL ; T. KISSEL.** Efficient and Tumor Targeted siRNA Delivery by Polyethylenimine-graft-polycaprolactone-block-poly (ethylene glycol)-folate (PEI-PCL-PEG-Fol). *Molecular pharmaceutics,* 2015, vol. 13, 134-143
- **Y. XIE ; N. KIM ; V. NADITHE ; D. SCHALK ; A. THAKUR ; A. KILIC ; D. BASSETT ; L. LUM ; O. MERKEL.** Targeted delivery of siRNA to activated T cells via transferrin-polythylenimine (Tf-PEI) as a potential therapy of asthma. *Journal of Controlled Release,* 2016, vol. 229, 120-129
- **HORIBA.** *Understanding the Chi Square and R Parameter - Calculations in the LA-950 Software,* 4, https://www.horiba.com/fileadmin/uploads/Scientific/Documents/PSA/TN1 53.pdf
- **S. CLAUS ; C. WEILER ; J. SCHIEWE ; W. FRIESS.** Optimization of the fine particle fraction of a lyophilized lysozyme formulation for dry powder inhalation. *Pharm. Res.,* 2013, vol. 30, 1698-1713
- **R. KANDIL ; Y. XIE ; R. HEERMANN ; L. ISERT ; K. JUNG ; A. MEHTA ; O.M. MERKEL.** Coming in and Finding Out: Blending Receptor-Targeted Delivery and Efficient Endosomal Escape in a Novel Bio-Responsive siRNA Delivery System for Gene Knockdown in Pulmonary T Cells. *Advanced therapeutics,* 2019, vol. 2, 1900047
- **Y.R. XIE ; N.H. KIM ; V. NADITHE ; D. SCHALK ; A. THAKUR ; A. KILIC ; L.G. LUM ; D.J.P. BASSETT ; O.M. MERKEL.** Targeted delivery of siRNA to activated T cells via transferrin-polyethylenimine (Tf-PEI) as a potential therapy of asthma. *J. Control. Release,* 2016, vol. 229, 120-129
- **BÜCHI-LABORTECHNIK-AG.** *Training Papers Spray Drying,* 1997, https://static1.buchi.com/sites/default/files/downloads/Set_3_Training_Papers_Spray_Drying_en_01.pdf?996b2db24007502bd69c913b675467cfc63 880ba
- **M. TERRAZAS ; E.T. KOOL.** RNA major groove modifications improve siRNA stability and biological activity. *Nucleic Acids Res.,* 2009, vol. 37, 346-353
- **D. WEINBUCH ; J.K. CHEUNG ; J. KETELAARS ; V. FILIPE ; A. HAWE ; J. DEN ENGELSMAN ; W. JISKOOT.** Nanoparticulate Impurities in Pharmaceutical-Grade Sugars and their Interference with Light Scattering-Based Analysis of Protein Formulations. *Pharm. Res,* 2015, vol. 32, 2419-2427
- **N.K. JAIN ; I. ROY.** Effect of trehalose on protein structure. *Protein Sci.,* 2009, vol. 18, 24-36
- **I. VOLLRATH ; W. FRIESS ; A. FREITAG ; A. HAWE ; G. WINTER.** Comparison of ice fog methods and monitoring of controlled nucleation success after freeze-drying. *Int. J. Pharm,* 2019, vol. 558, 18-28
- **J.H. GITTER ; R. GEIDOBLER ; I. PRESSER ; G. WINTER.** A comparison of controlled ice nucleation techniques for freeze-drying of a therapeutic antibody. *J. Pharm. Sci.,* 2018, vol. 107, 2748-2754
- **J.H. CROWE ; L.M. CROWE ; D. CHAPMAN.** Preservation of membranes in anhydrobiotic organisms: the role of trehalose. *Science,* 1984, vol. 223, 701-703
- **J.H. CROWE ; F.A. HOEKSTRA ; L.M. CROWE.** Anhydrobiosis. *Annu. Rev. Physiol.,* 1992, vol. 54, 579-599
- **K. IZUTSU ; S. KOJIMA.** Excipient crystallinity and its protein-structure-stabilizing effect during freeze-drying. *Journal of pharmacy and pharmacology,* 2002, vol. 54, 1033-1039
- **M. ADLER ; G. LEE.** Stability and surface activity of lactate dehydrogenase in spray-dried trehalose. *J. Pharm. Sci.,* 1999, vol. 88, 199-208
- **M. MAURY ; K. MURPHY ; S. KUMAR ; L. SHI ; G. LEE.** Effects of process variables on the powder yield of spray-dried trehalose on a laboratory spray-dryer. *Eur. J. Pharm. Biopharm.,* 2005, vol. 59, 565-573
- **N.Y. CHEW ; H.-K. CHAN.** Influence of particle size, air flow, and inhaler device on the dispersion of mannitol powders as aerosols. *Pharm. Res.,* 1999, vol. 16, 1098-1103
- **Y.Y. LEE ; J.X. WU ; M. YANG ; P.M. YOUNG ; F. VAN DEN BERG ; J. RANTANEN.** Particle size dependence of polymorphism in spray-dried mannitol. *Eur. J. Pharm. Sci,* 2011, vol. 44, 41-48

- **K.J. GEH ; M. HUBERT ; G. WINTER.** Progress in formulation development and sterilisation of freeze-dried oligodeoxynucleotide-loaded gelatine nanoparticles. *Eur. J. Pharm. Biopharm.,* 2018, vol. 129, 10-20
- **E.Y. SHALAEV ; G. ZOGRAFI.** How does residual water affect the solid-state degradation of drugs in the amorphous state?. *J. Pharm. Sci.,* 1996, vol. 85, 1137-1141
- **J.H. GITTER ; R. GEIDOBLER ; I. PRESSER ; G. WINTER.** Significant Drying Time Reduction Using Microwave- Assisted Freeze- Drying for a Monoclonal Antibody. *J. Pharm. Sci.,* 2018, vol. 107, 2538-2543
- **L. WENG ; S. ZIAEI ; G.D. ELLIOTT.** Effects of Water on Structure and Dynamics of Trehalose Glasses at Low Water Contents and its Relationship to Preservation Outcomes. *Sci. Rep.,* 2016, vol. 6, 28795
- **I. GONDA ; A.F. ABDELKHALIK.** On the Calculation of Aerodynamic Diameters of Fibers. *Aerosol Sci. Technol.,* 1985, vol. 4, 233-238
- **F. CARDARELLI ; L. DIGIACOMO ; C. MARCHINI ; A. AMICI ; F. SALOMONE ; G. FIUME ; A. ROSSETTA ; E. GRATTON ; D. POZZI ; G. CARACCIOLO.** The intracellular trafficking mechanism of Lipofectamine-based transfection reagents and its implication for gene delivery. *Sci. Rep.,* 2016, vol. 6, 25879
- **J.S. KIM ; T.J. YOON ; K.N. YU ; M.S. NOH ; M. WOO ; B.G. KIM ; K.H. LEE ; B.H. SOHN ; S.B. PARK ; J.K. LEE.** Cellular uptake of magnetic nanoparticle is mediated through energy-dependent endocytosis in A549 cells. *J. Vet. Sci.,* 2006, vol. 7, 321-326
- **Y. LIU ; O. SAMSONOVA ; B. SPROAT ; O. MERKEL ; T. KISSEL.** Biophysical characterization of hyper-branched polyethylenimine-graft-polycaprolactone-block-mono-methoxyl-poly (ethylene glycol) copolymers (hy-PEI-PCL-mPEG) for siRNA delivery. *J. Control. Release,* 2011, vol. 153, 262-268
- **A. RAY ; L. COHN.** Th2 cells and GATA-3 in asthma: new insights into the regulation of airway inflammation. *J. Clin. Invest.,* 1999, vol. 104, 985-993
- **S.M. HOY.** Patisiran: First Global Approval. *Drugs,* 2018, vol. 78, 1625-1631
- **R. KANDIL ; Y. XIE ; R. HEERMANN ; L. ISERT ; K. JUNG ; A. MEHTA ; O.M. MERKEL.** Coming in and Finding Out: Blending Receptor-Targeted Delivery and Efficient Endosomal Escape in a Novel Bio-Responsive siRNA Delivery System for Gene Knockdown in Pulmonary T Cells. *Adv. Ther. (Weinheim, Ger.),* 2019, vol. 2, 1900047
- **S. SCHÜLE ; W. FRIEß ; K. BECHTOLD-PETERS ; P. GARIDEL.** Conformational analysis of protein secondary structure during spray-drying of antibody/mannitol formulations. *Eur. J. Pharm. Biopharm.,* 2007, vol. 65, 1-9
- **C. GARCIA-GALAN ; O. BARBOSA ; R. FERNANDEZ-LAFUENTE.** Stabilization of the hexameric glutamate dehydrogenase from Escherichia coli by cations and polyethyleneimine. *Enzyme Microb Technol,* 2013, vol. 52, 211-217
- **J. HORN ; E. TOLARDO ; D. FISSORE ; W. FRIESS.** Crystallizing amino acids as bulking agents in freeze-drying. *Eur. J. Pharm. Biopharm.,* 2018, vol. 132, 70-82
- **R.E. JOHNSON ; C.F. KIRCHHOFF ; H.T. GAUD.** Mannitol-sucrose mixtures-versatile formulations for protein lyophilization. *J. Pharm. Sci.,* 2002, vol. 91, 914-922
- **A. HAWE ; W. FRIESS.** Physicochemical characterization of the freezing behavior of mannitol-human serum albumin formulations. *AAPS PharmSciTech,* 2006, vol. 7, 94